# EUROPEAN PATENT SPECIFICATION

(11) **EP 2 764 009 B1**
(45) Date of publication and mention of the grant of the patent: **06.12.2017**
(21) Application number: 12825032.1
(22) Date of filing: 21.08.2012
(51) Int. Cl.: C07K 7/08, A61K 38/10, A61K 38/16, C07K 14/54, A61P 3/00, A61P 19/02

(54) **PEPTIDES FOR USE IN THE TREATMENT OF IL-1 RELATED DISEASES AND CONDITIONS**
PEPTIDE ZUR VERWENDUNG BEI DER BEHANDLUNG VON IL-1-BEDINGTEN KRANKHEITEN UND LEIDEN
PEPTIDES POUR L'UTILISATION DANS LE TRAITEMENT DE MALADIES ET D'ÉTATS ASSOCIÉS À IL-1

(30) Priority: 22.08.2011 AU 2011903358; 22.08.2011 AU 2011903360
(43) Date of publication of application: 13.08.2014
(73) Proprietor: Paradigm Biopharmaceuticals Limited, Melbourne, Victoria 3000 (AU)
(72) Inventor: PASPALIARIS, Vasilis, Malvern East, Victoria 3145 (AU); LANGAN, Brett, Kensington, Victoria 3031 (AU)
(74) Representative: Boult Wade Tennant
(86) International application number: PCT/AU2012/000977
(87) International publication number: WO 2013/026089

(56) References cited:
- WO-A1-95/20973
- WO-A1-95/20973
- WO-A1-96/39165
- US-A1- 2006 094 663
- YANOFSKY S D ET AL: "HIGH AFFINITY TYPE I INTERLEUKIN 1 RECEPTOR ANTAGONISTS DISCOVERED BY SCREENING RECOMBINANT PEPTIDE LIBRARIES", PROCEEDINGS OF THE NATIONAL ACADEMY OF SCIENCES, NATIONAL ACADEMY OF SCIENCES, US, vol. 93, 1 July 1996 (1996-07-01), pages 7381-7386, XP000985913, ISSN: 0027-8424, DOI: 10.1073/PNAS.93.14.7381
- LOWMAN H B: "BACTERIOPHAGE DISPLAY AND DISCOVERY OF PEPTIDE LEADS FOR DRUG DEVELOPMENT", ANNUAL REVIEW OF BIOPHYSICS AND BIOMOLECULAR STRUCTURE, ANNUAL REVIEWS INC., PALO ALTO, CA, US, vol. 26, 1 January 1997 (1997-01-01), pages 401-424, XP002936628, ISSN: 1056-8700, DOI: 10.1146/ANNUREV.BIOPHYS.26.1.401
- WEEDEN, T. ET AL.: 'A retro-inverso alpha-melanocyte stimulating hormone analog with MC1R-binding selectivity' JOURNAL OF PEPTIDE SCIENCE vol. 17, 2011, pages 47 - 55, XP055146343
- LI, C. ET AL.: 'Limitations of peptide retro-inverso isomerization in molecular mimicry' JOURNAL OF BIOLOGICAL CHEMISTRY vol. 285, 2010, pages 19572 - 19581, XP055146350

## Description

### TECHNICAL FIELD

The present invention relates to a peptide, more particularly to a peptide comprising D-amino acids, and compositions containing the peptide, which are suitable for the treatment of IL-1 related diseases and conditions.

### BACKGROUND

In this specification where a document, act or item of knowledge is referred to or discussed, this reference or discussion is not an admission that the document, act or item of knowledge or any combination thereof was at the priority date, publicly available, known to the public, part of common general knowledge; or known to be relevant to an attempt to solve any problem with which this specification is concerned.

Interleukin 1 (IL-1) is a pluripotent cytokine responsible for a large range of normal physiological roles. Interleukin 1 (IL-1) exists in two forms; IL-1 alpha (IL-1α) and IL-1 beta (IL-β). Both are agonists of the IL-1 receptor (IL-1R) and are proinflammatory cytokines that are implicated in a variety of infectious responses as well as in nearly all inflammatory diseases including diseases having inflammatory components. The effect of IL-1 is mediated by binding of IL-1 to the interleukin-1 receptor (IL-1R). The receptor-ligand complex then binds IL-1R accessory protein, and the resulting receptor-heterodimer transduces a cellular signal.

IL-1 is responsible for normal physiological roles ranging from the induction of vascular permeability and fever during sepsis to the increased secretion of additional cytokines in autoimmune diseases. Other roles of IL-1 include production and release of prostaglandins, pituitary hormones, and collagenases. IL-1 also stimulates the immune system to boost production of lymphocytes.

Typically, cytokines such as IL-1 respond to perturbations in normal cell homeostasis, which may arise through disease or external challenge. However, increased levels of IL-1α production are associated with several autoimmune disorders, ischemia, and various cancers, therefore implicating IL-1α in many diseases. Therefore, an important balance must be maintained in the body, between the beneficial effects of IL-1 and potential harmful effects of IL-1 on the vascular, endocrine, connective tissue, immune, hematopoietic, and central nervous systems. Multiple control mechanisms exist in the body to moderate the IL-1R mediated response. These antagonistic mechanisms include a decoy receptor (IL-1R2), soluble receptor fragments, and an IL-1R antagonist (IL-1Ra).

IL-1Ra is a member of the IL-1 cytokine family. This protein competitively inhibits the activities of IL-1, IL-1α and IL-1β, and modulates a variety of IL-1 related immune and inflammatory responses.

A recombinant full length IL-1 Ra has been approved for treatment in patients with methotrexate resistant rheumatoid arthritis (Amgen: Kineret®-Anakinra) and the full recombinant molecule is being investigated as a potential novel therapeutic in inhibiting cancer angiogenesis. However, recombinant IL-1 Ra has a short half-life of approximately six hours. As a consequence, daily injections are used in patients with rheumatoid arthritis.

Recombinant IL-1 Ra is a relatively large protein. The large size and complex nature of such proteins limit their therapeutic application due to the difficulty in producing them as well as their stability, solubility and bioavailability.

Although the sequences of IL-1R from different species are available, as well as tertiary structure data showing the nature of cytokine/receptor interactions, this has not assisted in the identification of a small molecule or peptide inhibitors that are antagonists of IL-1R.

Accordingly, there is a need for a small molecule IL-1R antagonist that may be easily prepared. There is also a need for peptides having suitable solubility, bioavailability and stability for use in treatment of IL-1 related diseases and conditions.

### SUMMARY

The inventors have found a synthetic peptide that is an antagonist of IL-1R. The peptide, and compositions comprising the peptide, are suitable for the treatment of IL-1 related diseases and conditions.

The present invention provides a peptide comprising the sequence of general formula (III):

Z₁-X₂₀-DLeu-DPro-DLeu-DAla-DTyr-DPro-DGln-DTrp-DTyr-DGly-DPro-DThr-DTrp-DGlu-DPhe-X₂₁-Z₂ (SEQ ID NO:3) (III)

or salts, solvates or tautomers thereof, wherein X₂₀ and X₂₁ are each independently absent or one or more amino acids; and Z₁ and Z₂ are each independently absent or a protecting group.

The present invention further provides a peptide of the sequence of general formula (IV):

Z₁-DLeu-DPro-DLeu-DAla-DTyr-DPro-DGln-DTrp-DTyr-DGly-DPro-DThr-DTrp-DGlu-DPhe (SEQ ID NO:4) (IV)

or salts, solvates or tautomers thereof, optionally wherein Z₁ is an acetyl protecting group.

The peptide comprising a sequence of general formula (III) or (IV) may optionally include the groups X₂₀ and X₂₁, which provide one or more additional amino acids at the N-terminus or the C-terminus or both, of the peptide. X₂₀ and/or X₂₁ may represent the addition of between 1 and 40 additional amino acids at the N-terminus or the C-terminus or both, of the peptide. In some embodiments, X₂₀ and/or X₂₁ represent the addition of between 1 and 30, 1 and 20, 1 and 10, 1 and 5, 2 and 6, 3 and 8 or 2 and 4 additional amino acids at the N-terminus or the C-terminus or both, of the peptide. These additional amino acids may be natural or non-natural amino acids. The additional amino acids may be L-amino acids or D-amino acids.

The peptide comprising a sequence of general formula (III) or (IV) may be optionally protected by the protecting groups Z₁ and Z₂. Suitable protecting groups are known in the art and include those disclosed in Greene, T. W., "Protective Groups in Organic Synthesis" John Wiley & Sons, New York 1999, as are methods for their installation and removal.

The peptides of the present invention may be in the form of a free peptide, or in the form of a salt, solvate, derivative, isomer or tautomer thereof.

The peptides of the present invention may be peptidomimetics of recognized ligands of IL-1R, and are capable of modulating the effects of IL-1. In one embodiment, the present invention provides an IL-1 receptor antagonist comprising a peptide comprising the sequence of general formula (III) or (IV) or a salt, solvate or tautomer thereof as defined above. In another embodiment, the present invention provides a peptide comprising the sequence of general formula (III) or (IV) or a salt, solvate or tautomer thereof as described above for use in therapy. The peptide may be for use in inhibiting the effect of IL-1.

The present invention also provides an IL-1 receptor antagonist comprising a peptide comprising the sequence of general formula (III) or (IV) or a salt, solvate or tautomer thereof as defined above.

The present invention generally relates to peptides and compositions comprising the peptide, which are suitable for the treatment of IL-1 related diseases and conditions. Accordingly, the present invention provides a pharmaceutical composition comprising a peptide comprising the sequence of general formula (III) or (IV) or a salt, solvate or tautomer thereof as defined above and a pharmaceutically acceptable carrier. Preferably, the peptide will be present in an amount sufficient for a suitable dosage. The composition will generally contain an appropriate dosage level of the peptide, which may, for example, be about 0.01 µg per kg to about 20 mg per kg subject body weight per day which can be administered in a single dose or multiple doses.

The present invention generally relates to peptides and compositions comprising the peptide, which are suitable for the treatment of IL-1 related diseases or conditions including inflammation, autoimmune diseases and immune diseases or diseases or conditions comprising an inflammatory, autoimmune or immune component. In one aspect, the present invention provides a peptide comprising the sequence of general formula (III) or (IV) or a salt, solvate or tautomer thereof as described above, for use in treatment of IL-1 related diseases and conditions such as inflammation, autoimmune diseases and immune diseases or diseases or conditions comprising an inflammatory, autoimmune or immune component.

Preferably, the IL-1 related diseases or conditions are selected from inflammatory bowel disease, ulcerative colitis, distal colitis, Chrohn's disease, inflammation, equine laminitis, osteoarthritis, mastitis, pain and cachexia.

### DETAILED DESCRIPTION

The present invention generally relates to peptides and compositions comprising the peptide, which are suitable for the treatment of IL-1 related diseases and conditions such as inflammation, autoimmune diseases and immune diseases or diseases or conditions comprising an inflammatory, autoimmune or immune component.

Described herein is a peptide comprising a sequence of the general formula (I):

Z₁-X₂₀-DX₁-DX₂-DX₃-DX₄-DX₅-DX₆-DX₇-DX₈-DX₉-DX₁₀-DX₁₁-DX₁₂-DX₁₃-DX₁₄-DX₁₅-X₂₁-Z₂ (SEQ ID NO:1) (I)

or salts, solvates or tautomers thereof, wherein
X₁ is DLeu, DVal or DIle; X₂ is DPro or Aze; X₃ is DLeu, DVal or DIle; X₄ is DAla or DGly; X₅ is DTyr, DTrp, DPhe or DHis; X₆ is DPro or Aze; X₇ is DGln, DSer, DThr, DMet, DCys or DAsn; X₈ is DTrp, DTyr, DPhe or DHis; X₉ is DTyr, DTrp, DPhe or DHis; X₁₀ is DGly or DAla; X₁₁ is DPro or Aze; X₁₂ is DThr, DGl_{N}, DSer, DMet, DCys or DAsn; X₁₃ is DTrp, DTyr, DPhe or DHis; X₁₄ is DGlu or DAsp; X₁₅ is DPhe, DTyr, DTrp or DHis; X₂₀ and X₂₁ are each independently absent or one or more amino acids; and Z₁ and Z₂ are each independently absent or a protecting group.

The peptide comprising a sequence of general formula (I) may optionally include one or more additional amino acids at the N-terminus or the C-terminus or both, of the peptide. As indicated above, the groups X₂₀ and X₂₁ are each independently absent or one or more amino acids. When present, X₂₀ and/or X₂₁ each independently represent the addition of between 1 and 5 additional amino acids at the N-terminus or the C-terminus or both, of the peptide. In some embodiments, X₂₀ and/or X₂₁ represent the addition of between 2 and 4 additional amino acids at the N-terminus or the C-terminus or both, of the peptide. These additional amino acids may be natural or non-natural amino acids. The additional amino acids may be L-amino acids or D-amino acids.

Also described herein is a peptide comprising a sequence of general formula (II):

Z₁-X₂₀-DLeu-DPro-DLeu-DAla-DTyr-DX₆-DGln-DX₈-DX₉-DGly-DPro-DThr-DTrp-DGlu-DPhe-X₂₁-Z₂ (SEQ ID NO:2) (II)

or salts, solvates or tautomers thereof, wherein X₆ is DPro or Aze; X₈ is DTrp or DTyr; X₉ is DTyr or DTrp; X₂₀ and X₂₁ are each independently absent or between one and five amino acids; and Z₁ and Z₂ are each independently absent or a protecting group.

The present invention provides a peptide comprising a sequence of general formula (III):

Z₁₋X₂₀-DLeu-DPro-DLeu-DAla-DTyr-DPro-DGln-DTrp-DTyr-DGly-DPro-DThr-DTrp-DGlu-DPhe-X₂₁-Z₂ (SEQ ID NO:3) (III)

or salts, solvates or tautomers thereof, wherein X₂₀ and X₂₁ are each independently absent or one or more amino acids; and Z₁ and Z₂ are each independently absent or a protecting group.

In another embodiment, the present invention provides a peptide of the sequence of general formula (IV):

Z₁-DLeu-DPro-DLeu-DAla-DTyr-DPro-DGln-DTrp-DTyr-DGly-DPro-DThr-DTrp-DGlu-DPhe (SEQ ID NO:4) (IV)

or salts, solvates or tautomers thereof, optionally wherein Z₁ is an acetyl protecting group. The term "amino acid" is used herein in its broadest sense, unless otherwise specified, and includes L- and D-amino acids including L- and D-isomers of the 20 common amino acids namely alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine; and L- and D-isomers of the less common amino acid derivatives such as homo-amino acids (e.g. β-amino acids), *N*-alkyl amino acids, dehydroamino acids, aromatic amino acids and α,α-disubstituted amino acids, for example, cystine, 5-hydroxylysine, 4-hydroxyproline, α-aminoadipic acid, α-amino-*n*-butyric acid, 3,4-dihydroxyphenylalanine, homoserine, α-methylserine, ornithine, omithine, pipecolic acid, ortho, meta or para-aminobenzoic acid, citrulline, canavanine, norleucine, δ-glutamic acid, aminobutyric acid, fluorenylalanine, 3-benzothienylalanine and thyroxine; β-amino acids (as compared with the typical α-amino acids) and L- and D-isomers of any amino acid having a molecular weight less than about 500. The term amino acid also includes non-natural amino acids. Examples of non-natural amino acids include: Nle=L-norleucine; Aabu=aminobutyric acid; Hphe=L-homophenylalanine; Nva=L-norvaline; Dala=D-alanine; Dcys=D-cysteine; Dasp=D-aspartic acid; Dglu=D-glutamic acid; Dphe=D-phenylalanine; Dhis=D-histidine; Dile=D-isoleucine; Dlys=D-lysine; Dleu=D-leucine; Dmet=D-methionine; Dasn=D-asparagine; Dpro=D-proline; Dgln=D-glutamine; Darg=D-arginine; Dser=D-serine; Dthr=D-threonine; Dval=D-valine; Dtrp=D-tryptophan; Dtyr=D-tyrosine; Dorn=D-omithine; Aib=arninoisobutyric acid; Etg=L-ethylglycine; Tbug=L-t-butylglycine; Pen=penicillamine; Anap=I-naphthylalanine; Chexa=cyclohexylalanine; Cpen=cyclopentylalanine; Cpro=aminocyclopropane carboxylate; Norb=aminonorbomylcarboxylate; Mala=L-[alpha]-methylalarnine; Mcys=L-[alpha]-methylcysteine; Masp=L-.alpha.-methylaspartic acid; Mglu=L-[alpha]-methylglutamic acid; Mphe=L-[alpha]-methylphenylalanine; Mhis=L [alpha]-methylhistidine; Mile=L-[alpha]-methylisoleucine; Mlys=L-[alpha]-methyllysine; Mleu=L-[alpha]-methylleucine; Mmet=L-[alpha]-methylmethionine; Masn=L-[alpha]-methylasparagine; Mpro=L-[alpha]-methylproline; Mgln=L-[alpha]-methylglutamine; Marg=L-[alpha]-methylarginine; Mser=L-a-methylserine; Mthr=L-[alpha]-methylthreonine; Mval=L-a-methylvaline; Mtrp=L-[alpha]-methyltryptophan; Mtyr=L-a-methyltyrosine; Morn=L-[alpha]-methylomithine; Mnle=L-a-methylnorleucine; amino-[alpha]-methylbutyric acid;. Mnva=L-a-methylnorvaiine; Mhphe=L-[alpha]-methylhomophenylalanine; Metg=L-a-methylethylglycine; methyl-[gamma]-aminobutyric acid; methylaminoisobutyric acid; Mtbug=L-[alpha]-methyl-t-butylglycine; methylpenicillamine; methyl-[alpha]-naphthylalanine; methylcyclohexylalanine; methylcyclopentylalanine; Dmala=D-[alpha]-methylalanine; Dmorn=D-[alpha]-methylomithine; Dmcys=D-[alpha].-methylcysteine; Dmasp=D-[alpha]-methylaspartic acid; Dmglu=D-[alpha]-methylglutamic acid; Dmphe=D-[alpha]-methylphenylalanine; Dmhis=D-[alpha]-methylhistidine; Dmile=D-[alpha]-methylisoleucine; Dmlys=D-[alpha]-methyllysine; Dmleu=D-[alpha]-methylleucine; Dmmet=D-[alpha]-methylmethionine; Dmasn=D-[alpha]-methylasparagine; Dmpro=D-[alpha]-methylproline; Dmgln=D-[alpha]-methylglutamine; Dmarg=D-[alpha]-methylarginine; Dmser=D-[alpha]-methylserine; Dmthr=D-[alpha]-methylthreopine; Dmvai=D-[alpha]-methylvaline; Dmtrp=D-[alpha]-methyltryptophan; Dmtyr=D-[alpha]-methyltyrosine; Nmala=L-N-methylalanine; Nmcys=L-N-methylcysteine; Nmasp=L-N-methylaspartic acid; Nmglu=L-N-methylglutamic acid; Nmphe=L-N-methylphenylalanine; Nmhis=L-N-methylhistidine; Nmile=L-N-methylisoleucine; Nmlys=L-N-methyllysine; Nmleu=L-N-methylleucine; Nmmet=L-N-methylmethionine; Nmasn=L-N-methylasparagine; Nmchexa=N-methylcyclohexylalanine; Nmgln=L-N-methylglutamine; Nmarg=L-N-methylarginine; Nmser=L-N-methylserine; Nmthr=L-N-methylthreonine; Nmval=L-N-methylvaline; Nmtrp=L-N-methyltryptophan; Nmtyr=L-N-methyltyrosine; Nmorn=L-N-methylomithine; Nmnle=L-N-methylnorleucine; Nmaabu=N-amino-[alpha]-methylbutyric acid; Nmnva=L-N-methylnorvaline; Nmhphe=L-N-methylhomophenylalanine; Nmetg=L-N-methylethylglycine; Nmgabu=N-methyl-y-aminobutyric acid; Nmcpen=N-methylcyclopentylalanine; Nmtbug=L-N-methyl-t-butylglycine; Nmpen=N-methylpenicillamine; Nmanap=N-methyl-a-naphthylalanine; Nmaib=N-methylaminoisobutyric acid; Naeg=N-(2-aminoethyl)glycine; Dnmala=D-N-methylalanine; Dnmorn=D-N-methylomithine; Dnmcys=D-N-methylcysteine; Dnmasp=D-N-methylaspartic acid; Dnmglu=D-N-methylglutamic acid; Dnmphe=D-N-methylphenylalanine; Dnmhis=D-N-methylhistidine; Dnmile=D-N-methylisoleucine; Dnmlys=D-N-methyllysine; Dnmleu=D-N-methylleucine; Dnmmet=D-N-methylmethionine; Dnmasn=D-N-methylasparagine; Dnmpro=D-N-methylproline; Dnmgln=D-N-methylglutamine; Dnmarg=D-N-methylarginine; Dnmser=D-N-methylserine; Dnmthr=D-N-methylthreonine; Dnmval=D-N-methylvaline; Dnmtrp=D-N-methyltryptophan; Dnmtyr=D-N-methyltyrosine; Nala=N-methylglycine (sarcosine); Nasp=N-(carboxymethyl)glycine; Nglu=N-(2-carboxyethyl)glycine; Nphe=N-benzylglycine; Nhhis=N-(imidazolylethyl)glycine; Nile=N-(1-methylpropyl)glycine; Nlys=N-(4-aminobutyl)glycine; Nleu=N-(2methyylpropyl)glycine; Nmet=N-(2-methylthioethyl)glycine; Nhser=N-(hydroxyethyl)glycine; Nasn=N-(carbamylmethyl)glycine; Ngln=N-(2-carbamylethyl)glycine; Nval=N-(1-methylethyl)glycine; Narg=N-(3-guanidinopropyl)glycine; Nhtrp=N-(3-indolylethyl)glycine; Nhtyr=N-(p-hydroxyphenethyl)glycine; Nthr=N-(1-hydroxyethyl)glycine; Ncys=N-(thiomethyl)glycine; Norn=N-(3-aminopropyl)glycine; Ncpro=N-cyclopropylglycine; Ncbut=N-cyclobutyglycine; Nchex=N-cyclohexylglycine; Nchep=N-cycloheptylglycine; Ncoct=N-cyclooctylglycine; Ncdec=N-cyclodecylglycine; Ncund=N-cycloundecylglycine; Ncdod=N-cyclododecylglycine; Nbhm=N-(2,2-diphenylethyl)glycine; Nbhe=N-(3,3-diphenylpropyl)glycine; Nnbhm=N-(N-(2,2-diphenylethyl)carbamylmethyl)glycine; Nnbhe=N-(N-(3,3-diphenylpropyl)carbamylmethyl)glycine; and Nbmc=1-carboxy-1-(2,2-diphenylethylamino)cyclopropane. Azetidine-2-carboxylic acid (Aze or J) is another example of a non-natural amino acid that may be included in the peptide of the present invention.

The term "side chain" is used herein in the usual sense and refers to the side chain on the amino acid, and the backbone to the H₂N-(C)ₓ-CO₂H (where x = 1, 2 or 3) component, in which the carbon in bold text bears the side chain (the side chain being possibly linked to the amino nitrogen, as in the case of proline).

The amino acids may be optionally protected. The term "protected" is used herein in its broadest sense and refers to an introduced functionality which renders a particular functional group, such as a hydroxyl, thiol, amino, carbonyl or carboxyl group, unreactive under selected conditions and which may later be optionally removed to unmask the functional group. A protected amino acid is one in which the reactive substituents of the amino acid, the amino group, carboxyl group or side chain of the amino acid are protected. Suitable protecting groups are known in the art and include those disclosed in Greene, T. W., "Protective Groups in Organic Synthesis" John Wiley & Sons, New York 1999 as are methods for their installation and removal.

The amino group of the amino acid may be optionally protected. In one embodiment, the *N*-protecting group is an acyl or acetyl (Ac), carbamate such as, 9-fluorenylmethyl carbamate (Fmoc), 2,2,2-trichloroethyl carbamate (Troc), *t*-butyl carbamate (Boc), allyl carbamate (Alloc), 2-trimethylsilylethyl (Teoc) and benzyl carbamate (Cbz). Preferably, the *N*-protecting group is acetyl.

The carboxyl group of the amino acid may be optionally protected. The carboxyl protecting group is preferably an ester such as an alkyl ester, for example, methyl ester, ethyl ester, *t*-Bu ester or a benzyl ester. The carboxyl group may also be protected by amidation.

The side chain of the amino acid may be optionally protected. For example, the side chain carboxyl groups of aspartic acid, glutamic acid and α-aminoadipic acid may be esterified (for example as a C₁-C₆ alkyl ester), the side chain amino groups of lysine, ornithine and 5-hydroxylysine, may be converted to carbamates (for example as a C(=O)OC₁-C₆ alkyl or C(=O)OCH₂Ar aromatic carbamates) or imides such as pthalimide or succinimide, the hydroxyl groups of 5-hydroxylysine, 4-hydroxyproline, serine, threonine, tyrosine, 3,4-dihydroxyphenylalanine, homoserine, α-methylserine and thyroxine may be converted to ethers (for example a C₁-C₆ alkyl or a (C₁-C₆ alkyl)arylether) or esters (for example a C(=O)C₁-C₆ alkyl ester) and the thiol group of cysteine may be converted to thioethers (for example a C₁-C₆ alkyl thioether) or thioesters (for example a C(=O)C₁-C₆ alkyl thioester).

The term "peptide" is used herein refers to any sequence of amino acids, regardless of length, post-translation modification, or function. "Polypeptide", "peptide" and "protein" are used interchangeably herein. The peptides of the invention comprise the sequence of general formula (I), (II), (III) or (IV), and may also include any additions to the amino acid sequence. In some embodiments, the peptide of the present invention is of a length that permits the peptide to be produced in large quantities, and to have good solubility properties, stability and bioavailability. In one embodiment, the peptide of the invention is between 15 and 95 amino acids in length. In another embodiment, the peptide of the present invention may be between 15 and 55, 15 and 45, 15 and 35, 15 and 25, 15 and 23, 15 and 21, 15 and 19, 17 and 23, 17 and 21, 17 and 19, 16 and 19 or 18 and 30 amino acids in length.

The term "polypeptide" is used herein refers to an oligopeptide, peptide or protein. Where "polypeptide" is recited, this term refers to an amino acid sequence of a naturally-occurring protein molecule, "polypeptide" and like terms are not intended to limit the amino acid sequence to the complete sequence of the polypeptide, but instead is intended to also encompass biologically active variants thereof, including polypeptides having substantial sequence similarity or sequence identity relative to the amino acid sequences provided herein.

The peptides of the present invention may be described as peptidomimetics. The peptides of the present invention comprise a sequence of the general formula (III) or (IV) that mimic identically or closely, a naturally occurring peptide. In particular, the peptides of the present invention may be peptidomimetics of recognized ligands of IL-1R.

The sequence of the general formula (I), (II), (III) or (IV) comprises D-amino acids. Peptides comprising D-amino acids are attractive as drugs because D-amino acids and their corresponding peptides are less susceptible to degradation in the stomach or inside cells by proteolysis. Such peptides drugs can therefore be taken orally and are effective for a longer period of time. Peptides comprising D-amino acids are also easy to synthesize, when compared to many other drugs.

The peptides of the present invention may be in the form of a free peptide, or in the form of a salt, solvate or tautomer thereof.

The salts of the peptides may be pharmaceutically acceptable, but it will be appreciated that non-pharmaceutically acceptable salts also fall within the scope of the present invention, since these are useful as intermediates in the preparation of pharmaceutically acceptable salts. Examples of pharmaceutically acceptable salts include salts of pharmaceutically acceptable cations such as sodium, potassium, lithium, calcium, magnesium, barium, ammonium and alkylammonium; acid addition salts of pharmaceutically acceptable inorganic acids such as hydrochloric, orthophosphoric, sulphuric, phosphoric, nitric, carbonic, boric, sulfamic and hydrobromic acids; salts of pharmaceutically acceptable organic acids such as acetic, propionic, butyric, tartaric, maleic, hydroxymaleic, fumaric, citric, lactic, mucic, gluconic, benzoic, succinic, oxalic, phenylacetic, methanesulphonic, trihalomethanesulphonic, toluenesulphonic, benzenesulphonic, salicylic, sulphanilic, aspartic, glutamic, edetic, stearic, palmitic, oleic, lauric, pantothenic, tannic, ascorbic and valeric acids; or salts or complexes with pharmaceutically acceptable metal ions, including non-toxic alkali metal salts such as sodium and potassium salts, or non-toxic transition metal complexes such as zinc complexes.

In addition, some of the peptides may form solvates with water (such as hydrates or hemihydrates) or common organic solvents. Such solvates are encompassed within the scope of the invention.

The term "derivative" as used herein refers to any ester, amide, active metabolite, or analogue of the peptide of the present invention, which is not biologically or otherwise undesirable and induces the desired pharmacological and/or physiological effect. The term "derivative" also encompasses glycosylated forms of the peptide of the invention. Preferably the derivative is a pharmaceutically acceptable derivative.

The term "tautomer" as used herein is used in its broadest sense and includes peptides of the present invention which are capable of existing in a state of equilibrium between two isomeric forms. Such compounds may differ in the bond connecting two atoms or groups and the position of these atoms or groups in the compound.

The term "isomer" as used herein is used in its broadest sense and includes structural isomers, geometric isomers and stereoisomers. Other than the α-carbon stereogenic centres that provide the amino acids in the D-configuration as specified in the general formula (I), (II), (III) or (IV), the peptides described herein may include one or more further stereogenic centres, which may exist in two or more enantiomeric forms. These further stereogenic centres (namely, any stereogenic centres other than the α-carbons of the D-amino acids that are specified in the general formula (I), (II), (III) or (IV), may be synthesised, used or both synthesised and used as a purified enantiomer, as an enriched enantiomer or diastereomer, or as a mixture of any ratio of stereoisomers. It is however preferred that where the peptide is present as a mixture of stereoisomers, the mixture is enriched in the preferred stereoisomer.

In its broadest sense "enriched" means that the mixture contains more of the preferred isomer than of the other isomer. Preferably, an enriched mixture comprises greater than 50% of the preferred isomer, where the preferred isomer gives the desired level of potency and selectivity. More preferably, an enriched mixture comprises at least 60%, 70%, 80%, 90%, 95%, 97.5% or 99% of the preferred isomer. The peptide described herein which is enriched in the preferred isomer can either be obtained *via* a stereospecific reaction, stereoselective reaction, isomeric enrichment *via* separation processes, or a combination of all three approaches.

It must be noted that, as used in the specification, the singular forms "a", "an" and "the" include plural aspects unless the context clearly dictates otherwise. Thus, for example, reference to "a peptide" includes a single peptide, as well as two or more peptides, respectively; and so forth.

### Preparation of the peptides

Standard procedures for preparing synthetic peptides are well known in the art. Accordingly, a person skilled in the art may synthesise the peptides of the invention as defined in general formula (III) or (IV).

The peptides of the invention may be synthesized using liquid-phase peptide synthesis (as described in, for example, Bodanzsky, M., Principles of Peptide Synthesis, 2nd revised ed., Springer-Verlag (1988 and 1993)). The peptides of the invention may also be synthesized using the solid phase peptide synthesis (SPPS) (as described in, Merrifield, R., J. Am. Chem. Soc., (1963), 85(14), 2149-2154) or modifications of the SPPS approach. The peptides of the invention may be prepared by SPSS using automated peptide synthesizers, or by manual synthesis.

The peptides may be synthesized using appropriately protected L-amino acids, D-amino acids, non-natural amino acids and/or amino acid analogs. Protected amino acids may be used in peptide synthesis. A protected amino acid (as described above as optionally protected amino acids) includes one or more protecting groups on one or more of the reactive groups (i.e. on one or more of the amino group, the carboxyl group and/or the side chain of the amino acid). Preferably, the protecting group used in peptide synthesis is a t-butyldicarbonate (t-BOC) group or a fluorenylmethoxy carbonyl (FMOC) group.

The L-amino acids, D-amino acids, non-natural amino acids or amino acid analogs used to synthesise the peptides of the invention may be obtained from commercial sources or may be synthesized using methods known in the art.

Where the SPSS approach is used, the solid phase resin, may for example be 4-methylbenzhydrylamine (MBHA), 4-(oxymethyl)-phenylacetamido methyl or 4-(hydroxymethyl)phenoxymethyl-copoly(styrene-1 % divinylbenzene) (Wang resin), or any other suitable, commercially available resin.

### Composition

The invention also provides a composition comprising a peptide of the present invention as the active ingredient, and a carrier.

The term "composition" is intended to include the formulation of an active ingredient with conventional carriers, and also with encapsulating materials as the carrier, to give a capsule in which the active ingredient (with or without other carriers) is surrounded by the encapsulation carrier. A suitable carrier must be compatible with the peptide of the invention and the other ingredients of the composition, and must not be deleterious to a subject.

The compositions of the present invention may contain other therapeutic agents, and may be formulated, for example, by employing conventional solid or liquid vehicles or diluents, as well as suitable additives that are appropriate for the desired mode of administration (for example, excipients, binders, preservatives, stabilizers, flavours, gelling agents, solvents, solubilizers, buffers, lubricants, suspending agents, disintegrating agents, sweeteners, antioxidants, isotonic agents and the like) according to techniques such as those well known in the art of pharmaceutical formulation (see, for example, Remington: The Science and Practice of Pharmacy, 21st Ed., 2005, Lippincott Williams & Wilkins).

The composition includes those suitable for oral, rectal, nasal, topical (including buccal and sub-lingual), vaginal or parenteral (including intramuscular, sub-cutaneous and intravenous) administration or in a form suitable for administration by inhalation or insufflation. Where the peptide includes L-amino acids, compositions for oral administration may not be preferred.

The peptides of the invention, together with a carrier may thus be placed into the form of a composition or a unit dosage, and may be employed as a solid, such as a tablet or filled capsule, or a liquid such as a solution, suspension, emulsion, elixir, or capsule filled with the same, all for oral use, in the form of a suppository for rectal administration; or in the form of a sterile injectible solution for parenteral (including subcutaneous) use.

Such compositions and unit dosage forms thereof may comprise conventional ingredients in conventional proportions, with or without additional active ingredients, and such unit dosage forms may contain any suitable effective amount of the active ingredient commensurate with the intended daily dosage range to be employed.

The term "carrier" as used herein includes any diluents, excipients, adjuvants, additives, solvents, dispersion media, coatings, actives, agents, enhancers or ingredients which may be added to the peptide of the invention to form a composition suitable for administration to a subject.

For preparing compositions or unit dosage forms from the peptides of the present invention, pharmaceutically acceptable carriers can be either solid or liquid. Solid form preparations include powders, tablets, pills, capsules, cachets, suppositories, and dispensable granules. A solid carrier can be one or more substances which may also act as a diluent, flavouring agent, solubiliser, lubricant, suspending agent, binder, preservative, tablet disintegrating agent, or an encapsulating material.

Suitable carriers include magnesium carbonate, magnesium stearate, talc, sugar, lactose, pectin, dextrin, starch, gelatin, tragacanth, methylcellulose, sodium carboxymethylcellulose, a low melting wax, cocoa butter and the like. The term "preparation" is intended to include the formulation of the active ingredient with an encapsulating material as the carrier by providing a capsule in which the active ingredient, with or without carriers, is surrounded by a carrier, which is thus in association with it. Similarly, cachets and lozenges are included. Tablets, powders, capsules, pills, cachets, and lozenges can be used as solid forms suitable for oral administration.

Liquid form preparations include solutions, suspensions, and emulsions, for example, water or water-benzyl alcohol solutions. For example, parenteral injection liquid preparations can be formulated as solutions in aqueous polyethylene glycol solution. In one embodiment, the injectible formulation includes water or saline, benzyl alcohol and sodium bicarbonate.

Sterile liquid form compositions include sterile solutions, suspensions, emulsions, syrups and elixirs. The active ingredient can be dissolved or suspended in a pharmaceutically acceptable carrier, such as sterile water, sterile organic solvent or a mixture of both.

The compositions according to the present invention may thus be formulated for parenteral administration (for example, by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilisation from solution, for constitution with a suitable vehicle, for example, sterile, pyrogen-free water or saline, before use.

Compositions according to the present invention which are suitable for injectible use include sterile injectible solutions or dispersions, and sterile powders for the extemporaneous preparation of sterile injectible solutions. They should be stable under the conditions of manufacture and storage and may be preserved against oxidation and the contaminating action of microorganisms such as bacteria or fungi.

The carrier for the injectible solution or dispersion may be a solvent or dispersion medium and may contain any of the conventional solvent or carrier systems for the peptides of the invention, and may contain, for example, water, saline, ethanol, benzyl alcohol, polyol (for example, glycerol, propylene glycol and liquid polyethylene glycol and the like, or benzyl alcohol and sodium bicarbonate or benzyl alcohol and other pharmaceutically acceptable salts), suitable mixtures thereof, and vegetable oils.

Compositions suitable for injectible use may be delivered by any appropriate route including intravenous, intramuscular, intracerebral, intrathecal, epidural injection or infusion.

Sterile injectible solutions are prepared by incorporating the peptide of the invention in the required amount in the appropriate solvent with various other ingredients such as those enumerated above, as required, followed by filtered sterilization. Generally, dispersions are prepared by incorporating the various sterilised active ingredients into a sterile vehicle which contains the basic dispersion medium and the required other ingredients from those enumerated above. In the case of sterile powders for the preparation of sterile injectible solutions, preferred methods of preparation are vacuum drying or freeze-drying of a previously sterile-filtered solution of the active ingredient plus any additional desired ingredients.

When the active ingredients are suitably protected they may be orally administered, for example, with an inert or assimilable edible carrier, or they may be enclosed in hard or soft shell gelatin capsule, or they may be compressed into tablets, or they may be incorporated directly with the food of the diet. For oral therapeutic administration, the peptide of the invention may be incorporated with carriers and used in the form of ingestible tablets, buccal tablets, troches, capsules, elixirs, suspensions, syrups, wafers and the like. Preferably, peptides which include L-amino acids are not administered orally.

The amount of the peptide of the invention in therapeutically useful compositions should be sufficient that a suitable dosage will be obtained. The composition will generally contain an appropriate dosage level of the peptide, which may be about 0.01 µg per kg to about 20 mg per kg subject body weight per day. The composition will generally contain an appropriate dosage level of the peptide, which may be administered in a single dose or in multiple doses. This is discussed in more detail in the section describing dosage regimes, below.

The amount of the carrier that is present in the composition will depend on the type of composition and the mode by which that composition will be administered. The carrier may be present in the composition in an amount from 0 up to about 50.0%w/w, from 0 up to about 60.0%w/w, from 0 up to about 80.0%w/w, from 0 up to about 90%w/w, from 0 up to about 99.0%w/w, or from 0 up to about 99.5%w/w of the total weight of the pharmaceutical composition. In some embodiments, the carrier is present in an amount of about 80.0%w/w, about 90%w/w, about 98%w/w, about 99%w/w, or about 99.5%w/w, of the total concentration of the composition.

The tablets, troches, pills, capsules and the like may also contain the components as listed hereafter:
- a binder such as gum, acacia, corn starch or gelatin;
- excipients such as dicalcium phosphate;
- a disintegrating agent such as corn starch, potato starch, alginic acid and the like;
- a lubricant such as magnesium stearate; and
- a sweetening agent such as sucrose, lactose or saccharin; or a flavouring agent such as peppermint, oil of wintergreen, or cherry flavouring.

When the unit dosage form is a capsule, it may contain, in addition to materials of the above type, a liquid carrier.

Various other materials may be present as coatings or to otherwise modify the physical form of the dosage unit. For instance, tablets, pills, or capsules may be coated with shellac, sugar or both. A syrup or elixir may contain the active ingredient, sucrose as a sweetening agent, methyl and propylparabens as preservatives, a dye and flavouring such as cherry or orange flavour. Of course, any material used in preparing any unit dosage form should be pure according to pharmaceutical and/or veterinary standards, and substantially non-toxic in the amounts employed. In addition, the peptide of the invention may be incorporated into sustained-release preparations and formulations, including those that allow specific delivery of the active ingredient to specific regions of the gut. For example, the peptide of the invention may be incorporated into an oral dosage form which is enterically coated.

Aqueous solutions suitable for oral use can be prepared by dissolving the active ingredient in water and adding suitable colorants, flavours, stabilising and thickening agents, as desired. Aqueous suspensions suitable for oral use can be made by dispersing the finely divided active ingredient in water with viscous material, such as natural or synthetic gums, resins, methylcellulose, sodium carboxymethylcellulose, or other well known suspending agents. Preferably, peptides which include L-amino acids are not administered orally.

Pharmaceutically acceptable carriers include any and all solvents, dispersion media, coatings, antibacterial and antifungal agents, isotonic and absorption delaying agents and the like.

Also included are solid form preparations that are intended to be converted, shortly before use, to liquid form preparations for oral administration. Such liquid forms include solutions, suspensions, and emulsions. These preparations may contain, in addition to the active ingredient, colorants, flavours, stabilisers, buffers, artificial and natural sweeteners, dispersants, thickeners, solubilising agents and the like.

For topical administration to the epidermis the compounds according to the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

When the composition is for topical or oral use, the composition may also comprise one or more agents known to accelerate the delivery of the peptide of the present invention through the skin or mucosa (including intestinal mucosa) of animals, including humans, which are sometimes known as penetration enhancers, accelerants, adjuvants, and sorption promoters, and are collectively referred to herein as "enhancers".

Compositions suitable for topical administration in the mouth include lozenges comprising active ingredient in a flavoured base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multidose form. In the latter case of a dropper or pipette, this may be achieved by the patient administering an appropriate, predetermined volume of the solution or suspension.

In the case of a spray, this may be achieved for example by means of a metering atomising spray pump. To improve nasal delivery and retention the peptides according to the invention may be encapsulated with cyclodextrins, or formulated with other agents expected to enhance delivery and retention in the nasal mucosa.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, a hydrofluorocarbon (HFC) for example hydrofluoroalkanes (HFA), carbon dioxide, or other suitable gas.

The aerosol may conveniently also contain a surfactant such as lecithin. The dose of drug may be controlled by provision of a metered valve.

Alternatively the active ingredient may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, for example gelatin, or blister packs from which the powder may be administered by means of an inhaler.

In formulations intended for administration to the respiratory tract, including intranasal formulations, the compound will generally have a small particle size for example of the order of 5 to 10 microns or less. Such a particle size may be obtained by means known in the art, for example by micronisation.

Liquids or powders for intranasal administration, tablets or capsules for oral administration and liquids for intravenous administration are the preferred compositions.

When desired, formulations adapted to give sustained release of the active ingredient may be employed.

The compositions are preferably in unit dosage forms. In such form, the preparation is subdivided into unit doses containing appropriate quantities of the active ingredient. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

It is especially advantageous to formulate parenteral compositions in unit dosage form for ease of administration and uniformity of dosage. Unit dosage form as used herein refers to physically discrete units suited as unitary dosages for the subjects to be treated; each unit containing a predetermined quantity of active ingredient calculated to produce the desired therapeutic effect in association with the required carrier. The specification for the unit dosage forms of the invention are dictated by and directly dependent on (a) the unique characteristics of the active ingredient and the particular therapeutic effect to be achieved, and (b) the limitations inherent in the art of compounding such an active ingredient for the treatment of IL-1 related diseases or conditions in living subjects having a diseased condition in which bodily health is impaired.

In one embodiment, the present invention provides an IL-1 receptor antagonist comprising a peptide comprising the sequence of general formula (III) or (IV) or a salt, solvate or tautomer thereof as described above.

The IL-1 receptor antagonist comprising a peptide comprising the sequence of general formula (III) or (IV) or a salt, solvate or tautomer thereof may be in the absence of carrier. The IL-1 receptor antagonist comprising a peptide comprising the sequence of general formula (III) or (IV) or a salt, solvate or tautomer thereof may be in unit dosage form.

### Preparation of compositions

The compositions of the present invention may be prepared by a variety of techniques.

One method of preparing the composition defined above comprises the step of combining the peptide of the invention with a carrier, in suitable quantities. The peptide may be combined with the carrier by mixing, and is preferably mixed until complete homogenisation is achieved. Preferably, the peptide and the carrier are agitated over an extended period of time, such as an hour or longer. Agitation may be conducted at room temperature, or at reduced temperatures, preferably between about 4°C and about 25°C.

Any excipients may be added with the carrier or after the peptide is combined with the carrier.

### Treatment of IL-1 related diseases or conditions

The peptides and compositions of the present invention are suitable for the treatment of IL-1 related diseases or conditions.

The "subject" may be mammal including humans and animals. In one embodiment, the mammal is a human, female or male. In other embodiments, the mammal is of the subclass Theria including the subclasses Metatheria, Eutheria and Prototheria.

Under the subclass of Metatheria are the superorders of Ameridelphia and Australidelphia which are inclusive of marsupials such as kangaroos and possums. The subclass of Eutheria is inclusive of placentals. Under the subclass of Eutheria are a number of superorders, which in turn encompass orders. The superorder of Xenarthra includes the orders Cingulata (armadillos) and Pilosa (anteaters and sloths). The superorder of Afrotheria includes the orders Afrosoricida (tenrecs and golden moles), Macroscelidea (elephant shrews), Tubulidentata (aardvarks), Hyracoidea (hyraxes), Proboscidea (elephants) and Sirenia (dugongs and manatees). The superorder of Laurasiatheria includes the orders Soricomorpha (shrews, moles and solenodons), Erinaceomorpha (hedgehogs and relatives), Chiroptera (bats), Pholidota (pangolins), Carnivora (dogs, cats, weasels, bears, seals, and their relatives), Perissodactyla (odd-toed ungulates), Artiodactyla (even-toed ungulates) and Cetacea (whales and dolphins), the latter two orders are sometimes referred to the order of Cetartiodactyla. The superorder of Euarchontoglires includes the orders Rodentia (mice, rats, porcupines, beavers, capybaras, and other gnawing mammals), Lagomorpha (rabbits and relatives), Scandentia (treeshrews), Dermoptera (colugos) and Primates (humans, apes and monkeys).

In one embodiment, the subject may be selected from humans, domestic mammals such as companion animals, working animals, livestock, and zoological/park mammals. Preferably, the subject is a human.

In some embodiments, the subject is a mammal, such as an ungulate (even-toed and odd-toed), such as a cow, goat, sheep, yak, water buffalo, horse, reindeer, camel, alpaca, banteng, donkey, oxen, zebu, moose or bison. The mammal may also be a domestic mammal including livestock, companion animal, working animal, or zoological/park mammal such as an ape, monkey, lion, zebra, and so on.

The peptide of the present invention functions as an IL-1 R antagonist. As such, the peptide of the present invention is suitable for the treatment of IL-1 related diseases or conditions. The term IL-1 related disease or condition includes any disease or condition involving IL-1 mediated activity having an adverse effect on a subject. In some IL-1 related diseases or conditions the levels of interleukin-1 (IL-1) will be elevated and/or levels of its natural antagonist IL-1 ra will be reduced. As an example, elevated levels of IL-1 and reduced levels of IL-1 ra have been observed in patients with inflammatory bowel disease.

In one embodiment, the IL-1 related disease or condition includes inflammation, autoimmune diseases or immune diseases. Diseases or conditions comprising an inflammatory, autoimmune or immune component are also considered to be IL-1 related diseases or conditions which may be treated using the peptides of the present invention. For example, cachexia is a metabolic syndrome in which the subject develops abnormal blood biochemistry including increased inflammatory markers and as such, cachexia is considered to be a disease or condition comprising an inflammatory component.

Examples of IL-1 related diseases or conditions include, but are not limited to, acne vulgaris, acute and chronic lung disease (including acute and chronic interstitial lung disease and inflammatory lung disease), acute febrile illness due to bacteria or viruses, acute myocardial infaction, alcoholic cirrhosis, anorexia, asthma, atherlerosclerosis, arthritis (for example rheumatoid arthritis, arthritis chronica progrediente, arthritis deformans, juvenile arthritis and spondylarthritis) and rheumatic diseases, asbestosis, autoimmune diseases (such as autoimmune haematological disorders, autoimmune cardiomyopathy, autoimmune hepatitis, autoimmune pancreatitis, autoimmune polyendocrine syndrome, autoimmune progesterone dermatitis, autoimmune thrombocytopenic purpura, autoimmune urticaria and autoimmune uveitis), autoimmune inflammatory bowel disease (including e.g. ulcerative colitis, distal colitis and Crohn's disease), bacterial infection, burns, cachexia, cancer, cardiopulmonary bypass, celiac disease, cerebral infarction (such as cerebral palsy), chronic active hepatitis, chronic prostatitis, chronic renal failure, coal miner pneumonoconiosis, contact dermatitis, cuprophane hemodialysis, dermatitis, dermatomyositis, diabetes (including juvenile diabetes, insulin diabetes, diabetes mellitus and type I diabetes), endocrine ophthalmopathy, eosinophilic fasciitis, equine laminitis, glomerulonephritis, gout and pseudogout, graft versus host disease, Graves disease, Guillain-Barré syndrome, Hashimoto's encephalitis, Hashimoto's thyroiditis, hemophagocytic lymphohistiocytosis, HIV infection and AIDS, hypersensitivities, idiopathic sprue, inflammatory bowel disease, interstitial lung fibrosis, ischemia injury, Kawasaki's disease, leukemia (including myelogenous leukemia), lupus erythematosus, luteal phase defect, Miller-Fisher syndrome, mixed connective tissue disease, keratoconjunctivitis sicca and vernal keratoconjunctivitis, mastitis, multiple sclerosis, myasthenia gravis, obstructive jaundice, osteomalacia, osteoarthritis, osteoporosis, pain, Paget's disease, pediatric sepsis syndrome, pelvic inflammatory disease, polychondritis, polymyositis, pre-eclampsia, primary biliary cirrhosis uveitis (anterior and posterior), psoriasis, psoriatic arthritis, pulmonary hypertension, relapsing polychondritis, reperfusion injury, respiratory distress syndrome, reticulohistiocytosis, sarcoidosis, scleroderma, sepsis, septic shock, Sjögren's syndrome, silicosis, Steven-Johnson syndrome, systemic lupus erythematosus, temporal arteritis, thermal injury, toxic shock syndrome, transplant rejection, tuberculosis, undifferentiated connective tissue disease, vasculitis and intestinal cystitis and Wegener granulamatosis. In one embodiment, the methods or uses involving the peptide comprising the sequence of general formula (I), (II), (III) or (IV) or a salt, solvate, derivative, isomer or tautomer thereof are for the treatment of IL-1 related diseases or conditions such as inflammatory bowel disease, ulcerative colitis, distal colitis, Chrohn's disease, inflammation, equine laminitis, osteoarthritis, mastitis, pain and cachexia.

Generally, the term "treating" means affecting a subject, tissue or cell to obtain a desired pharmacological and/or physiological effect and includes: (a) binding to and/or acting as an antagonist of IL-1R; (b) relieving or ameliorating the effects of IL-1 related diseases or conditions; (c) reducing the incidence of the IL-1 related diseases or conditions, or (d) preventing the IL-1 related diseases or conditions from occurring in a subject, tissue or cell predisposed to the IL-1 related disease or condition, or at risk thereof (but not yet been diagnosed with), by providing a protective pharmacological and/or physiological effect so that the IL-1 related disease or condition does not develop or occur.

Although the present invention has been described with reference to treating IL-1 related diseases or conditions such as inflammation, autoimmune diseases and immune diseases, it will be appreciated that the present invention may also be useful in the treatment of other diseases or disorders that are not specified, but are associated with inhibiting the effects of IL-1 and/or acting as an antagonist of IL-1R.

### Antagonising IL-1R

The peptide and the compositions of the present invention have been shown to function as antagonists of IL-1R, and therefore the peptide and compositions of the present invention provide an effective treatment of IL-1 related diseases or conditions such as inflammation, autoimmune diseases and immune diseases or diseases or conditions comprising an inflammatory, autoimmune or immune component.

Accordingly, described herein is a method for inhibiting the effect of IL-1 comprising administering a peptide comprising the sequence of general formula (I), (II), (III) or (IV) or a salt, solvate, derivative, isomer or tautomer thereof as defined above, to a subject in need thereof.

The present invention also provides a peptide comprising the sequence of general formula (III) or (IV) or a salt, solvate or tautomer thereof as defined above for use in therapy. The peptide may be for use in inhibiting the effect of IL-1.

By virtue of its ability to inhibit the effects of IL-1, the peptide of the present invention is useful in the treatment of IL-1 related diseases or conditions.

In one embodiment, the present invention provides a peptide comprising the sequence of general formula (III) or (IV) or a salt, solvate or tautomer thereof as defined above, for use in the treatment of IL-1 related diseases or conditions such as inflammation, autoimmune diseases and immune diseases or diseases or conditions comprising an inflammatory, autoimmune or immune component. Also described herein is use of a peptide comprising the sequence of general formula (I), (II), (III) or (IV) or a salt, solvate, derivative, isomer or tautomer thereof as defined above, in the manufacture of a medicament for the treatment of inflammation, autoimmune diseases and immune diseases or diseases or conditions comprising an inflammatory, autoimmune or immune component. In one embodiment, the IL-1 related diseases or conditions are selected from inflammatory bowel disease, ulcerative colitis, distal colitis, Chrohn's disease, inflammation, equine laminitis, osteoarthritis, mastitis, pain and cachexia.

Administration of the peptide or composition to a subject may reduce the effects of IL-1 related diseases or conditions by up to 90%, up to 80%, up to 70%, up to 60%, up to 50%, up to 40%, up to 30%, up to 20%, or up to 10%. The reduction may be achieved in 1 week, 2 weeks, 3 weeks, 4 weeks, 5 weeks, or more, with daily or weekly administration of an effective dose of the peptide or the composition to a subject.

### Administration route

Routes of administration can broadly be divided into a three categories by effect, namely, "topical" where the desired effect is local, so the substance is applied directly where its action is desired, "enteral" where the desired effect is systemic (non-local) so the substance is given *via* the digestive tract, and "parenteral" where the desired effect is systemic, so the substance is given by routes other than the digestive tract.

The peptide or composition of the present invention is suitable for topical, enteral or parenteral administration.

Examples of topical routes of administration having a local effect include epicutaneous (onto the skin).

Examples of enteral routes of administration having a systemic (non-local) effect include any form of administration that involves any part of the gastrointestinal tract, such as oral (into the mouth), intranasal (into the nose), rectal (into the rectum), and vaginal (into the vagina). Oral administration includes buccal administration (absorbed through the cheek near the gumline) and sublingual administration (under the tongue).

Examples of parenteral routes of administration by injection, infusion or diffusion having a systemic effect include intravenous (into a vein), intraarterial (into an artery), intramuscular (into a muscle), intracardiac (into the heart), subcutaneous (under the skin), percutaneous (via needle-puncture into the skin), intradermal (into the skin itself), intrathecal (into the spinal canal), intraperitoneal (infusion or injection into the peritoneum), intravesical infusion (infusion into the urinary bladder), epidural (injection or infusion into the epidural space), transdermal or transcutaneous (diffusion through the intact skin), transmucosal (diffusion through a mucous membrane), insufflation (diffusion through the nose), inhalational (diffusion through the mouth), sublingual (under the tongue), and buccal (absorbed through cheek near gumline).

Preferably, the peptide or composition of the present invention is administered subcutaneously, intravenously, intramuscularly, orally, sublingually, transdermally, or by inhalation. Preferably, peptides which include L-amino acids are not administered orally.

### Dosage form

The peptide or composition of the present invention may be prepared into any suitable dosage form for topical, enteral, parenteral administration.

A person skilled in the art would readily appreciate what would be a suitable dosage form for topical, enteral, parenteral administration.

Suitable dosage forms for topical administration include creams, lotions, gels and the like. The dosage form may also be a patch or other device.

Suitable dosage forms for enteral administration would include but not be limited to capsules, tablets, pills, or specialty tablets such as buccal, sublingual, chewable tablets or orally-disintegrating tablets. Another example of a suitable dosage form would be edible thin films.

Other suitable dosage forms for enteral administration include liquid solutions or suspensions. Suitable liquid solution or suspension dosage forms may be in the form of a drink, such as sports drinks containing electrolytes (e.g. gatorade), or syrup and elixirs. Other suitable liquid solution or suspension dosage forms include nasal delivery solutions and oral suspensions including liquid solutions or suspensions. For veterinary purposes, the liquid solution or suspension may be in the form of a "drench". The liquid solution or suspension may also be used in the preparation of an edible product, such as a biscuit or cake, suitable for human or animal consumption. A product for animal consumption may also include appropriate animal feeds.

The dosage form for enteral administration may also be a powder or solid crystal, which can be either dissolved or suspended in a liquid before administration. Alternatively, the powder may be consumed directly or added to a food or drink product for consumption. In the case of farm animals, the formulation may be added directly to the animal feed.

Where the composition or the formulation has a disagreeable taste, additives with sufficient flavour to disguise the bad taste may be added to the dosage form (e.g. masking agents).

Examples of suitable dosage forms for parenteral administration include but are not limited to injectibles (i.e. solutions, suspensions, emulsions, and dry powders for reconstitution), intramammary infusions, intravaginal delivery systems, and implants.

### Dosage regime

The term "therapeutically effective amount" refers to the amount of a peptide or composition of the present invention that will elicit the biological or medical response of a subject, tissue or cell that is being sought by the researcher, veterinarian, medical doctor or other clinician. Administration of a "therapeutically effective amount" of the peptide or composition of the present invention should obtain a desired pharmacological and/or physiological effect which includes: (a) binding to and/or acting as an antagonist of IL-1R; (b) relieving or ameliorating the effects of IL-1 related diseases or conditions; (c) reducing the incidence of IL-1 related diseases or conditions, or (d) preventing the IL-1 related diseases or conditions from occurring in a subject, tissue or cell predisposed to the IL-1 related disease or condition, or at risk thereof (but not yet diagnosed with), by providing a protective pharmacological and/or physiological effect so that the IL-1 related disease or condition does not develop or occur.

It will be understood that the specific dose level and frequency of dosage for any particular subject may be varied and will depend upon a variety of factors including the activity of the peptide or composition employed, and the metabolic stability and length of action of that peptide or composition; the age, body weight, general health, sex, or diet of the subject; the route of administration used; the mode and time of administration of the peptide or composition; the rate of excretion or clearance of the peptide or composition from the body; the peptide used in the composition; the severity of the inflammation or infection to be treated; and the particular subject undergoing treatment.

Suitable intervals of dosing include monthly, every two months or longer, biweekly, weekly, daily, or multiple times per day. Preferably, the peptide or composition of the present invention is administered weekly, daily, or multiple times per day.

The dosage form will generally contain an appropriate dosage level of the peptide, which may be about 0.01 µg per kg to about 20 mg per kg subject body weight per day which can be administered in single or multiple doses. In some embodiments, the dosage level will be about 0.01 µg per kg to about 15 mg/kg per day; about 0.01 µg per kg to about 10 mg/kg per day; about 0.01 µg per kg to about 10 mg/kg per day, about 0.01 µg per kg to about 5 mg/kg per day, about 0.01 µg per kg to about 2.5 mg/kg per day or about 0.01 µg per kg to about 2 mg/kg per day.

A suitable dosage level may be about 0.005 µg per kg to about 10 mg/kg per day. In one embodiment, the dosage level may be about 0.01 µg per kg to about 5 mg per kg subject body weight per day which can be administered in single or multiple doses.

Within the above dosage ranges, the dosage may be about 0.1 mg/kg per day, about 0.2 mg/kg per day, about 0.4 mg/kg per day, about 0.6 mg/kg per day, about 0.7 mg/kg per day, about 0.8 mg/kg per day, about 0.9 mg/kg per day, about 1 mg/kg per day, about 1.2 mg/kg per day, about 1.4 mg/kg per day, about 1.5 mg/kg per day, about 1.6 mg/kg per day, about 1.7 mg/kg per day, about 1.8 mg/kg per day, about 1.9 mg/kg per day, about 2 mg/kg per day, about 2.2 mg/kg per day, about 2.5 mg/kg per day, about 3 mg/kg per day, about 5 mg/kg per day, about 7.5 mg/kg per day or about 10 mg/kg per day. In another embodiment, the dosage may be about 0.1 to about 15 mg/kg, or about 0.5 mg to about 2 mg. The dosage may vary depending on the subject to which the peptide of the invention is administered. For example, where the subject weighs about 50 to 100kg (such as a human subject), the dosage level of the peptide may be about 0.1 to 2 mg which corresponds to about 0.001 mg/kg to about 0.04 mg/kg. As another example, where the subject weighs about 400-600kg (such as an animal subject), the dosage level of the peptide may be about 0.1 to 2 mg which corresponds to about 0.17 µg/kg to about 5 µg/kg.

Preferably, the peptide or composition will be administered daily and will comprise the peptide in an amount of about 0.01 to about 1 mg/kg.

An effective dosage form comprises the peptide in the above defined amounts.

### Advantages

It has been surprisingly found that the peptides of the general formula (I), (II), (III) or (IV) have advantages in comparison to the corresponding L-enantiomers and other sequences. For example, the peptides of general formula (I), (II), (III) or (IV) generally show higher potency in blocking IL-1R on human monocytes and higher potency in blocking a human monocyte inflammatory response *in vitro.* By way of comparison, a peptide of the present invention has a lower IC₅₀ value and thus higher potency in blocking IL-1R on human monocytes compared to its corresponding L-enantiomer, while also having a significantly lower IC₅₀ value and higher potency in blocking a human monocyte inflammatory response *in vitro.*

It has also been surprisingly found that the peptides of the general formula (I), (II), (III) or (IV) are well tolerated and safe in advanced cancer patients and may be used in the management of patients with advanced neoplastic disease and cancer-related cachexia. For example, the peptides of general formula (I), (II), (III) or (IV) generally induce statistically significant improvement in anorexia, physical performance, and depression in patients with cancer-related cachexia.

The present inventors have also found that the peptides of the general formula (I), (II), (III) or (IV) may be used in the treatment of patients with inflammatory bowel disease, ulcerative colitis, distal colitis and Chrohn's disease. For example, the peptides of general formula (I), (II), (III) or (IV) generally show a reduction in IL-1α-induced IL-8 production in HT 29 and Caco2 colonic epithelial cell lines. The peptides also show a reduction in IL-1α and IFN-γ-induced IL-8 or IL-17 production.

In addition, the peptides of the present invention are generally more soluble and easier to dissolve than the corresponding L-enantiomers. This is coupled with a longer half-life in human plasma, meaning that the peptides of the present invention can be administered in smaller quantities and/or less frequently. Furthermore, the peptides of the present invention have been shown to be non-toxic at therapeutic threshold in mice. These properties all contribute to providing the peptides with advantageous properties in comparison to the corresponding L-enantiomers and other sequences.

### FIGURES

Example 2 is described with reference to the accompanying figures in which:
Figure 1 is a chart showing the reduction in IL-1α-induced IL-8 production by the peptide of sequence 4 at 100ng/ml and 500ng/ml compared to control experiments when tested on HT29 epithelial cells.
Figure 2 is a chart showing the reduction in IL-1α-induced IL-8 production by IL-1 ra at 50ng/ml, 100ng/ml and 500ng/ml compared to the peptide of sequence 4 at 100ng/ml and control experiments when tested on Caco2 colonic adenocarcinoma cells.
Figure 3 is a chart showing the reduction in IL-1α and IFN-γ induced IL-8 production by the peptide of sequence 4 at concentrations from 5ng/ml to 1µg/ml compared to control experiments when tested on HT29 cells.
Figure 4 is a chart showing the reduction in IL-1α and IFN-γ induced IL-17 production by the peptide of sequence 4 at concentrations of 10ng/ml and 500ng/ml compared to control experiments when tested in HT29 cells.

### EXAMPLES

The invention is described further by reference to the following non-limiting examples of the invention.

### Example 1: Comparative testing of In vitro Interleukin 1 binding and in vitro inhibition of Interleukin-1 stimulation of Interleukin-8

Tests were conducted to compare the properties of 18 peptides. The sequences of these peptides are provided in Table 1 below. Sequences 5, 7, 9 and 11 are deduced from the natural human IL-1Ra amino acid sequence. The peptide sequences 13, 15, 17, 19 and 20 were identified from peptide display libraries as described in Yanofsky, S.D. et al., "High affinity type I interleukin 1 receptor antagonists discovered by screening recombinant peptide libraries", Proc. Natl. Acad. Sci. USA, (1996) Vol. 93, pp. 7381-7386.

The peptides of sequences 5, 7, 9, 11, 13, 15, 17, 19 and 20 are composed of L-amino acids, and have an acetyl group on the N-terminal amino acid. The peptides of sequences 6, 8, 10, 12, 14, 16, 18, 4 and 21 are retro-inverso peptidomimetics of the peptides of sequences 5, 7, 9, 11, 13, 15, 17, 19 and 20, respectively. Accordingly, the peptides of sequences 6, 8, 10, 12, 14, 16, 18, 4 and 21 are composed of D-amino acids arranged in the reverse order to sequences 5, 7, 9, 11, 13, 15, 17, 19 and 20. These peptides also have an acetyl group on the N-terminal amino acid.

**TABLE 1: Peptide sequences that have been tested.**

| **SEQ ID NO.** | **Amino Acid Sequence** | **Amino Acid Type** |
|---|---|---|
| 5 | Ac-NNQLVAGYLQGPNVNLEEKIDVVPIEPHALFLGIHGGK | L-amino acids |
| 6 | Ac-KGGHIGLFLAHPEIPVVDIKEELNVNPGQLYGAVLQNN | D-amino acids |
| 7 | Ac-NNQLVAGYLQGPNVN | L-amino acids |
| 8 | Ac-NVNPGQLYGAVLQNN | D-amino acids |
| 9 | Ac-IDVVPIEAPHALFLGIHGGK | L-amino acids |
| 10 | Ac-KGGHIGLFLAHPAEIPVVDI | D-amino acids |
| 11 | Ac-LQLEAVNITDLSENRKQDK | L-amino acids |
| 12 | Ac-KDQKRNESLDTINVAELQL | D-amino acids |
| 13 | Ac-ETPFTWEESNAYYWQPYALPL | L-amino acids |
| 14 | Ac-LPLAYPQWYYANSEEWTFPTE | D-amino acids |
| 15 | Ac-FTWEESNAYYWQPYALPL | L-amino acids |
| 16 | Ac-LPLAYPQWYYANSEEWTF | D-amino acids |
| 17 | Ac-TANVSSFEWTPGYWQPYALPL | L-amino acids |
| 18 | Ac-LPLAYPQWYGPTWEFSSVNAT | D-amino acids |
| 19 | Ac FEWTPGYWQPYALPL | L-amino acids |
| 4 | Ac-LPLAYPQWYGPTWEF | D-amino acids |
| 20 | Ac-DGYDRWRQSGERYWQPYALPL | L-amino acids |
| 21 | Ac-LPLAYPQWYREGSQRWRDYGD | D-amino acids |

Each of the 18 peptides was tested to determine the *in vitro* binding affinity of each peptide for IL-1R.

Radioligand competition binding assays were performed in 96-well plates containing confluent human monocytes, which express the IL-1 receptor. Wells were washed three times with binding buffer, and binding buffer was placed in each well. Peptides were resuspended in dimethyl sulfoxide at 20 mM and diluted in binding buffer to the desired starting concentration. Using a Beckman robotic system, five 3-fold dilutions were prepared, and peptide solution was transferred to duplicate receptor-containing wells. To each well, radioactive ¹²⁵Iodine-labeled IL-la (Amersham) was added to a final concentration of 40 pM, and the plates were placed on a shaking platform at 4° C for 2 hr. Plates were washed with PBS using a plate washer, and bound ¹²⁵I-labeled IL-la was eluted for 10 min with 0.1 M NaOH and counted in a gamma counter. IC₅₀ values were determined by fitting the data to a two-parameter logistic equation. In all cases, nonspecific binding was determined by the addition of 3 µM of recombinant IL-la.

Inhibition of IL-1 induced IL-8 in human fibroblasts was determined using normal human dermal fibroblast cells cultured in DMEM with 10% fetal calf serum in 48-well plates. Twenty-four hours before initiation of the experiment, the medium was changed to DMEM with 0.2% fetal calf serum. Each peptide was added to confluent cells, which were then pulsed for 30 min with rIL-I,B (which was obtained from R & D Systems) at 50 pg/ml. The cells were washed four times with PBS, and fresh DMEM/0.2% fetal calf serum was added to each well. After 5 hr of incubation, the culture media were collected and analyzed by ELISA for IL-8 (which was obtained from R & D Systems). Maximal activation, with IL-1 only, was 2530 ± 13 pg/ml of IL-8. Nonspecific activation, determined in the presence of 1000-fold excess IL-1 Ra, was 30 ± 9 pg/ml of IL-8 (n = 3).

Table 2 shows IC₅₀ values measured in IL-1 *in vitro* binding assays on human monocytes, and in *in vitro* inhibition of IL-1 stimulation of Interleukin-8 (IL-8) in human skin fibroblasts. The IC₅₀ values shown in this table are an average value of three separate measurements and a standard error of the three independent tests.

**TABLE 2: In vitro IL-1 binding antagonism on human monocytes, and in vitro inhibition of IL-1 stimulation of IL-8 in human skin fibroblasts.**

| **SEQ ID NO.** | **Binding (IC₅₀ nM)** | **Binding (IC50 nM)** |
|---|---|---|
| 5 | >10000 | ND |
| 6 | ND | ND |
| 7 | >10000 | ND |
| 8 | ND | ND |
| 9 | >10000 | ND |
| 10 | ND | ND |
| 11 | >10000 | ND |
| 12 | ND | >10000 |
| 13 | 11 + 3.1 | 11 + 2.1 |
| 14 | 9.2 + 2.7 | 11 + 1.4 |
| 15 | 20 + 5.2 | 16 + 1.8 |
| 16 | 22 + 6.1 | 120 + 13 |
| 17 | 7.6 + 1.9 | 76 + 12 |
| 18 | 190 + 19 | 810 + 180 |
| 19 | 7.1 + 2.1 | 10 + 1.5 |
| 4 | 6.9 + 0.5 | 5.9 + 0.9 |
| 20 | 8.1 + 1.3 | 19 + 2.2 |
| 21 | 240 + 21 | 730 + 160 |

The radioligand competition binding assay results show the comparison of the binding of the peptides on the IL1R, and the IL-1 stimulation of IL-8 assay results show the comparison of the various peptides on the inhibition of IL-1 driven cellular responses. These results show that by comparison, peptide 4 shows the highest antagonism of the IL-1R and the highest inhibition of IL-1 driven cellular responses.

### Example 2: In vitro effect of IL-1 ra on production of soluble inflammatory mediators by colonic epithelial cells intestinal myofibroblasts and colonic tissue from IBD patients.

### Experimental protocol

Colonic epithelial cells, intestinal myofibroblasts and colonic tissue from IBD patients and normal controls were used (as described in Kouroumalis, A, et al. (2005). J Immunol, 175, 5403-5411, Jordan, NJ, et al. (1999). J Clin Invest, 104, 1061-1069, Bourikas, LA, et al. (2009). Br J Pharmacol, 157, 362-370, and Weaver, SA, et al. (2001) Gastroenterology, 120, 1117-1127). Cell and tissue cultures were stimulated with vehicle controls or various doses of IL-1α added alone or in combination with other pro-inflammatory cytokines (TNF-α, IFN-γ) in the presence or absence of the peptide of sequence 4 or IL-1 ra, in a dose and time dependent manner.

The production of inflammatory mediators (e.g cytokines, chemokines, nitric oxide) and fibrotic agents (e.g tissue factor, CTGF, collagen) was assessed in supernatants, cell protein extractions and RNA extractions at the mRNA and protein level, (as described in Kambas, K, et al. (2011). J Immunol, 186, 6568-6575, Kolios, G, et al. (1999). Eur J Immunol, 29, 530-536, Kolios, G, et al. (1998). Gut, 43, 56-63 and Chrysanthopoulou, A, *et al.* (2011)). These experiments demonstrate the *in vitro* anti-inflammatory and possible anti-fibrotic effect of the peptides of the invention in intestinal inflammation.

### Methods

### Colon adenocarcinoma cell lines-cell cultures

Human colon epithelial cell lines HT-29 and Caco2 obtained from the European Collection of Animal Cell Cultures (ECACC) were used in cell cultures. Cells were routinely cultured at 37°C in an atmosphere of 5 % CO₂ in 80cm² tissue culture flasks in McCoy's and DMEM medium, respectively, supplemented with penicillin (10u/ml), streptomycin (10µg/ml), fungizone (0.5µg/ml), and 10 % (v/v) FCS (referred to as complete medium) until confluent.

Confluent monolayers were sub-cultured into 6-well plates for experimental protocols. Cells were grown in 6-well plates until confluent. Twenty-four hours prior to the experiment the confluent monolayers were washed and cultured in FCS-free McCoy's medium. Growth arrested cultures were then treated with fresh FCS-free medium and stimulated with vehicle controls or various doses of IL-1α added alone or in combination with other pro-inflammatory cytokines (TNF-α, IFN-γ) in the presence or absence of the peptide of sequence 4 or IL-1 ra, in a dose and time dependent manner.

Supernatants were collected, centrifuged to remove cellular debris and stored at -70°C until assayed for extracellular inflammatory mediators. Total RNA for RT-PCR was extracted as described above. Cell counting and viability was routinely checked at the beginning and the end of the experiment by phase microscopy and by trypan blue exclusion using representative wells. Mycoplasma tests were performed during the experimental protocol. Production of soluble mediators was assessed by commercially available ELISA in supernatants, RT-PCR in total RNA extractants and nitric oxide, as described in Kolios, G, et al. (1995). Br J Pharmacol, 116, 2866-2872.

### Colonic biopsies

Multiple colonic biopsies were taken from patients, who underwent colonoscopy at the University Hospital of Alexandroupolis. The study included: (1) biopsies from inflamed mucosa of patients with a recent clinical diagnosis of ulcerative colitis but had yet to start on any treatment, (2) biopsies from histologically normal mucosa of the proximal colon from those of the above patients with distal colitis, and (3) biopsies from histologically normal mucosa of patients with no colonic disease who underwent diagnostic colonoscopy. An experienced consultant histopathologist with a special interest in gastrointestinal pathology, unaware of which patients are included in any experiments, established the diagnosis. Consent was obtained from patients in all cases and the local research ethics committee granted approval for the study.

### Biopsy culture

Biopsies were cultured as were described in Linehan, JD, et al. (2005a). Am J Physiol Gastrointest Liver Physiol, 288, G261-G267 and Linehan, JD, et al. (2005b). Free Radic Biol Med, 39, 1560-1569. Colonic biopsies were immediately placed in a transport medium of Hank's balanced salts solution, pH 7.4, supplemented with antibiotics. Colonic biopsies from inflamed tissue were incubated at 37°C in an atmosphere of 5% CO₂ in the presence or absence of the peptide of sequence 4 or IL-1 ra for a period of up to 30 hours. Colonic biopsies from normal tissue will be incubated at 37°C in an atmosphere of 5% CO₂ with vehicle, as controls, or with IL-1α added alone or in combination with proinflammatory cytokines in the presence or absence of the peptide of sequence 4 or IL-1 ra.

Supernatants were then collected for inflammatory mediator measurement and colonic specimens were used for RNA extraction and/or estimation of total protein per well. Inflammatory mediator production was estimated at the mRNA level by RT-PCR and at the protein level by ELISA, as described in Kolios, G, et al. (1999). Eur J Immunol, 29, 530-536 and Kolios, G, et al. (1998). Gut, 43, 56-63. Estimation of total protein per well was performed using the Bio-Rad Protein Microassay as described in Bradford, MM. (1976). Anal Biochem, 72, 248-254, and individual nitrite contents of each well were expressed as pmol/100 Ag of total protein.

### Colonic Subepithelial Myofibroblast Isolation

Studies were performed on subepithelial myofibroblasts (SEMFs; passages 3-8) isolated from normal colonic tissue. Tissue was obtained by endoscopic biopsy of apparently normal mucosa from patients according to rules set by our Institutional Review Board. Patients were included in the study after informed written consent prior to participation.

Colonic SEMFs were isolated as described in Kouroumalis, A, et al. (2005). J Immunol, 175, 5403-5411 and Kambas, K, et al. (2011). J Immunol, 186, 6568-6575. Mucosal specimens were collected in ice-cold Hank's balanced salt solution (HBSS) with Ca++/Mg++ plus penicillin (100 U/ml), streptomycin (100 µg/ml), amphotericin B (2.5 µg/ml), gentamicin (50 µg/ml), vancomycin (32 µg/ml) (GIBCO, UK) and de-epithelialised with three individual 10 minute treatments with 1 mM DTT (Sigma-Aldrich, US) along with three separate 30 minute incubations in 3 mM EDTA at 37°C. HBSS without Ca++/Mg++ plus the same antibiotics were used for wash and DTT- or EDTA-containing media.

Colonic tissue, denuded from epithelium, was ultimately incubated in Dulbecco's Modified Eagle Medium (DMEM, 4.5 g/L glucose, GIBCO, UK) plus 10% foetal bovine serum (FBS) and penicillin/streptomycin/amphotericin B (P/S/A) in 5% CO₂ at 37°C. During culturing, numerous non-adherent and adherent cells appeared in the culture flasks. The cells in suspension were removed every 72 hours, and the denuded mucosal tissue was maintained in culture for up to 4 weeks, until numerous foci of myofibroblasts were attached to the bottom of the culture flask.

Tissue specimens were removed, and intestinal myofibroblasts were cultured in DMEM supplemented with 10% FBS and P/S/A. The myofibroblast phenotype was verified using immunofluorescence microscopy by confirming the expression of smooth muscle actin and vimentin and the absence of desmin expression, as described in Kouroumalis, A, et al. (2005). J Immunol, 175, 5403-5411 and Kambas, K, et al. (2011). J Immunol, 186, 6568-6575.

### Colonic Subepithelial Myofibroblast Culture

Myofibroblasts from human colonic tissue were isolated as described above, and were stimulated with proinflammatory cytokines (IL-1, TNF, interferon gamma) in the presence or absence of the peptide of sequence 4 or IL-1 ra and inflammatory mediators. Expression as well as pro-collagen I and pro-collagen III production was examined in myofibroblasts derived from IBD mucosa (with and without presence of granulomas) in comparison to healthy controls. Collagen gene expression by myofibroblasts derived from fibrotic regions was compared to those derived from non-fibrotic regions. In addition each source of fibroblasts was stimulated with pro-inflammatory cytokines (TNF, IFN-γ, or TGF-β) added alone or in combination with IL-1α in the presence of the peptide of sequence 4, IL-1 ra or isotype matched control antibody in order to determine the time course and dose-response curve for pro-collagen I and III expression in cell extracts by RT-PCR or collagen production in supernatants, as described below. These experiments investigated whether the peptide of sequence 4 or IL-1 ra inhibits inflammatory mediators and pro-collagen gene expression in myofibroblasts and whether the peptide of sequence 4 or IL-1 ra modulates collagen production responsible for intestinal fibrosis in Crohn's disease.

### Total Intracellular Collagen Measurement

Cell lysates were added to Sirius Red dye (Sircol; Biocolor, UK), which bound to the side chain groups of the basic amino acids present in collagen. After 30 minutes incubation, the mixtures were centrifuged at 10,000 g for 10 minutes. The supernatant was discarded, and the collagen pellet was dissolved in 0.5 M NaOH alkali reagent for 10 minutes. Next, the ODs of the samples and controls of known concentration were measured at 540 nm, and the collagen concentration was calculated using a linear standard curve according to the manufacturer's instructions.

### Wound Healing Scratch Assay

Myofibroblast migration was assessed *in vitro* with the wound-healing scratch assay as described in Di, SA, et al. (2009). Gut, 58, 777-789 and Liang, CC, et al. (2007) Nat Protoc, 2, 329-333. A narrow gap devoid of cells was created on the SEMF cultures. The rate of gap closure was measured. This process resembles wound healing and is dependent on myofibroblast migration as described in Liang, CC, et al. (2007). Nat Protoc, 2, 329-333.

The FBS concentration in the media was gradually reduced from 10% to 5% to minimise the contribution of proliferation. Subsequently, monolayers of confluent SEMF cultures in 6-well plates were given one mechanical wound per well by scoring with a 200-µl pipette tip. Wounds were vertical to pre-drawn lines on the bottom of the well.

Images were recorded at fixed wound points (more than 12 per well) with an inverted DMIRE2 Leica microscope equipped with a DFC300 FX Leica camera. SEMFs were treated, in the presence or absence of the peptide of sequence 4 or IL-1 ra, with IL-1α added alone or in combination with pro-inflammatory cytokines (TNF-α, IFN-γ, TGF-β1) or ECC media from epithelial cells that were pre-treated in the absence or presence of pro-inflammatory cytokines in the presence or absence of IL-1 ra. Wells were reassessed at 48 hours.

The average percentage of gap closure after 48 hours in treated wells was divided by the average percentage of gap closure in untreated wells to assess the treatment effect (as described in Chrysanthopoulou, A, et al. (2011). Arthritis Rheum, 63, 3586-3597). These experiments examined the role of IL-1 ra in wound healing and its possible inhibitory effect in intestinal fibrosis.

### Colonic epithelial cells and myofibroblasts co-culture

Colonic myofibroblasts isolated from fibrotic and non-fibrotic gut wall were cultured with colonic cell lines in transwell chambers so that the cells are separated by a semi-permeable membrane, (as described in Omenetti, A, et al. (2011). Am J Physiol Gastrointest Liver Physiol, 300, G303-G315; Huang, M, et al. (2010). J Exp Clin Cancer Res, 29, 80; and Waghray, M, et al. (2005). FASEB J, 19, 854-856). Each side of the transwell was stimulated with IL-1 added alone or in combination with proinflammatory cytokines (TNF-α, IFN-γ, or TGF-β) to investigate whether the myofibroblasts or epithelial cells "talk" to each other via the semi-permeable membrane when stimulated. The use of microbeads prevented movement of stimuli between each side of the transwell.

Expression and production of collagen, and inflammatory mediators (such as cytokines, chemokines, nitric oxide) was monitored on either side of the transwell, using the techniques described above. These experiments investigated whether epithelial cells modulate collagen gene expression by myofibroblasts and whether the peptide of sequence 4 or IL-1 ra has an inhibitory role in this process that leads to intestinal fibrosis in IBD.

### Results

### The effect of IL-1ra and peptide 4 on IL-1α induced IL-8 production by colonic epithelial cells

HT 29 and Caco2 colonic epithelial cell lines were stimulated with 10 ng/ml of IL-1α in the presence of 100 or 500 ng/ml of the peptide of sequence 4.

Untreated HT-29 cells or cells stimulated with the peptide solvent (Control) did not result in production of IL-8, nor did cells treated with IL-1 ra in the absence of IL-1α (IL-1 ra 500 control). In contrast, the addition of IL-1α induced significant production of IL-8 (67.455 ± 0.125 ng/ml per 106 cells, n=6).

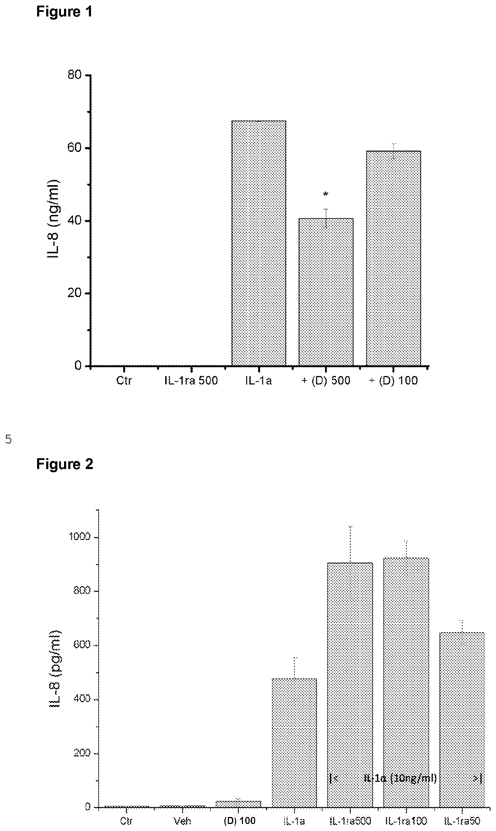

The presence of the peptide of sequence 4 demonstrated a different pattern. The peptide of sequence 4 reduced IL-1α-induced IL-8 production in a concentration dependent manner as shown in Figure 1. Significant inhibition was observed when the peptide of sequence 4 was at a concentration of 500 ng/ml (40.705 ± 2.56 ng/ml per 106 cells, n=6, p<0.01).

To further examine the effect of the peptide of sequence 4 on production by HT-29 cells, further experiments were conducted using a broad range of concentrations for the peptide of sequence 4 (5ng/ml-1µg/ml). The peptide of sequence 4 was found to continue to show a concentration dependent response as shown in Figure 1.

Similar experiments were conducted using Caco2 colonic adenocarcinoma cells. Unstimulated cells or cells stimulated with the peptide solvent did not result in production of IL-8 (Control). Caco2 cells stimulated with IL-1α secreted IL-8 (476.93 ± 78.9 pg/ml per 106 cells, n=3). IL-1 ra induced a significant increase up to 923.95 ± 63.69 pg/ml per 106 cells, n=3 at the concentration of 100 ng/ml, while the peptide of sequence 4, at a concentration of 100 ng/ml, induced a significant decrease as shown in Figure 2.

### The effect of IL-1ra and peptide 4 on IL-1α + IFN-γ induced IL-8 production by HT-29 cells

Stimulation of HT-29 cells with a combination of pro-inflammatory cytokines induces increased secretion of chemokines, including IL-8 (as described in Kolios, G, et al. (1999). EurJ Immunol, 29, 530-536). To examine the effect of the peptide of sequence 4 on co-stimulation of HT-29 cells with IL-1α and other pro-inflammatory cytokines, HT-29 cells were treated with a combination of 10ng/ml IL-1α + 300U IFN-γ in the presence of various concentrations (5 ng/ml-1µg/ml) of the peptide of sequence 4.

Untreated HT-29 cells (Control) or cells stimulated with the peptide solvent (Vehicle) did not result in production of IL-8, nor did cells treated with IL-1 ra in the absence of IL-1α (IL-1 ra control) or cells treated with IFN-γ in the absence of IL-1α (IFN-γ control).

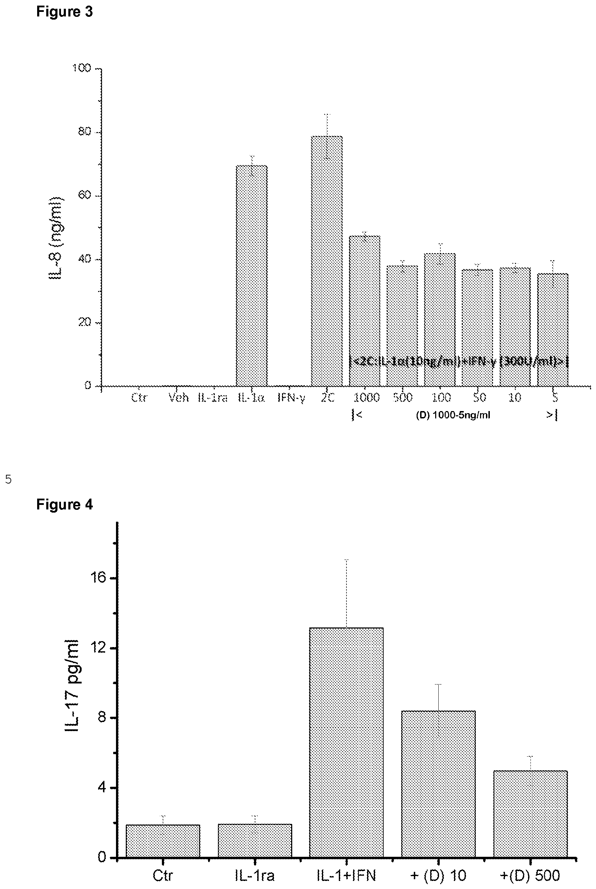

IFN-γ was found to have a synergistic effect with IL-1α on the IL-8 production increasing the IL-1α induced IL-8 production increasing the IL-8 production in HT-29 cells from 67.49 ± 3.12 ng/ml per 106 cells, n=3 (IL-1α control) to 78.79 ± 6.99 ng/ml per 106 cells, n=3 (2C). Pre-treatment of HT-29 with the peptide of sequence 4 prior to stimulation with IL-1α + IFN-γ demonstrated a permanent significant (p<0.01) inhibitory effect for all concentrations used, even at low concentrations (5 ng/ml), 35.41 ± 4.23 ng/ml per 106 cells, n=3, as shown in Figure 3.

### The effect of IL-1ra and peptide 4 on IL-1α + IFN-γ induced IL-17 production by HT-29 cells

To examine a possible immunoregulatory effect of the peptide of sequence 4 on the intestinal inflammation the effect of both peptides on the IL-17 production was examined. HT-29 cells were treated with the combination of 10ng/ml IL-1α + 300U IFN-γ in the presence of of the peptide of sequence 4 (10 ng/ml and 500 ng/ml).

Untreated HT-29 cells (Control) or cells stimulated with the IL-1ra peptide (IL-1 ra control) did not produce significant amounts of IL-17. In contrast the combination of 10ng/ml IL-1α + 300U IFN-γ induced a significant IL-17 production (13.14 ± 3.92 pg/ml per 106 cells, n=6) (IL-1 + IFN-γ). Interestingly, the presence of the peptide of sequence 4 significantly (p<0.001) reduced this IL-1α + IFN-γ-induced IL-17 production (as shown in Figure 4) to 4.97 ± 0.84 pg/ml per 106 cells, n=6 for the peptide of sequence 4.

### Example 3: Comparative testing of the solubility of peptides 13, 14, 19 and 4

Each of the peptides having the sequences 13, 14, 19 and 4 were tested to determine their solubility under different solvent conditions.

Table 3 shows the results obtained when the solubility of each of the peptides having the sequences 13, 14, 19 and 4 were tested in hydrochloric acid (0.1-1%), sodium bicarbonate (0.1-1%), benzyl alcohol (0.1-1%) and methanol (1-10%). 100mg of each peptide was added to 100ml total of water plus the solvents, and the solution was gently agitated for 30 min at room temperature.

In table 3, peptide solubility is described as follows: "Yes" indicates that the peptide completely dissolved, "No" indicates that the peptide did not dissolve at all, "+" indicates that the peptide was slightly soluble, "++" indicates that the peptide was moderately soluble and "ND" indicates that the test was not determined.

**TABLE 3: Solubility of peptide.**

| **SEQ ID NO.** | **Acid** | **Base** | **Benzyl Alcohol** | **Methanol** |
|---|---|---|---|---|
| 13 | No | + | No | Yes |
| 14 | No | + | No | Yes |
| 19 | No | ++ | Yes | ND |
| 4 | No | ++ | Yes | ND |

These results show that none of the peptides dissolved in acid alone, while the peptides were partially soluble in the presence of a base. Complete dissolution was observed with the initial addition of a base and then a solvent such as benzyl alcohol (peptide 19 and 4) or methanol (peptide 13 and 14). Accordingly, the peptides having the sequences 19 and 4 can be dissolved, and solutions can be prepared in water, with sodium bicarbonate (0.8%) and benzyl alcohol (0.3%). Such solutions would be suitable for parenteral administration.

### Example 4: Comparative testing of the stability of peptides 13, 14, 19 and 4

Each of the peptides having the sequences 13, 14, 19 and 4 were tested to determine their stability in a solution containing (preferred composition above) water, bicarbonate and either benzyl alcohol (peptide 19 and 4) or methanol (peptide 13 and 14). Each of the peptides were held for a period of 1 month at either 4°C or at room temperature.

Table 4 shows the results obtained when the stability of each of the peptides having the sequences 13, 14, 19 and 4 were tested in a solution containing a total of 5ml of water, bicarbonate and either benzyl alcohol or methanol. 1 mg of each peptide was added to the water, bicarbonate and either benzyl alcohol or methanol solution and the solution was gently agitated for 1 hour at room temperature before standing at either 4°C or at room temperature for a period of 1 month.

**TABLE 4: Stability of peptide once dissolved in solution water and methanol (at 1 month)**

| **SEQ ID NO.** | **4°C(month)** | **Room Temp(month)** |
|---|---|---|
| 13 | 21% | 1% |
| 14 | 78% | 18% |
| 19 | 95% | 48% |
| 4 | 100% | 96% |

These results show that peptide 4 has the highest stability of all of the peptides tested.

Each of the peptides having the sequences 13, 14, 19 and 4 were also tested to determine their stability in human plasma.

Table 5 shows the results obtained when the stability of each of the peptides having the sequences 13, 14, 19 and 4 were tested in human plasma. 1 mg of each peptide was added to the plasma and the solution was gently agitated for 2 minutes at room temperature before standing at 37°C for a period of 12 hours.

**TABLE 5: Human Plasma stability trials.**

| **SEQ ID NO.** | **½ Life human plasma (minutes)** |
|---|---|
| 13 | 46 |
| 14 | 333 |
| 19 | 49 |
| 4 | 1083 |

These results show that the half-life in human plasma was increased in both peptides 14 and 4, each of which are retro-inverso peptides containing D-amino acids. Peptide 4 performed the best having a half-life that was more than three-fold longer than that of peptide 14.

### Example 5: Comparative testing of the effect of peptides 13, 14, 19 and 4 on equine laminitis

Each of the peptides having the sequences 13, 14, 19 and 4 were also tested to determine their effect on equine laminitis. Each peptide was administered intravenously to a thoroughbred having equine laminitis. The peptides were prepared as compositions containing 1 mg of peptide dissolved in 5ml of a solution comprising water, bicarbonate and either benzyl alcohol or methanol. The effect of the peptides on equine laminitis was judged by the Obel Lameness Grades, which is a system of rating degrees of lameness resulting from laminitis. Obel Grade I features include frequent shifting of weight between the feet, no discernible lameness at the walk, and bilateral lameness at the trot. Obel Grade II horses do not resist having a foreleg lifted, nor are they reluctant to walk, but they do show lameness at the walk. Obel Grade II horses do resist having a foreleg lifted, and are reluctant to walk. Obel Grade IV horses will walk only if forced.

The Obel Score for each of the horses was measured before administration of the peptide and 24 hours after injection of the peptide.

Table 6 shows the change in the Obel scores measured before administration of the peptide and 24 hours after injection of the peptide, with the most negative number representing the greatest improvement in the horses health.

**TABLE 6: Effect of peptide candidates on Equine Laminitis.**

| **SEQ ID NO.** | **Change in Obel Score** |
|---|---|
| 13 | -0.5 |
| 14 | -1.1 |
| 19 | -2.1 |
| 4 | -2.9 |

These results show that peptide 19 and, to a greater extent, peptide 4, both had a marked effect in reducing laminitis. The effect in reducing laminitis which was caused by one single injection of the peptides on laminitis was short-lived. Therefore, the duration of the effect of the peptides in reducing laminitis was further assessed by attending veterinarians. Table 7 summarises these results.

**TABLE 7: Report by veterinarians is that the duration of effect of above peptide candidates on laminitis after a single 1 mg intravenous route averaged as follows:**

| **SEQ ID NO.** | **Duration (days)** |
|---|---|
| 13 | 1 |
| 14 | 2 |
| 19 | 2 |
| 4 | 4 |

These results show that the duration of the positive effect seen on equine laminitis by a single injection of peptide 4 was more than double that observed for the other peptides. **Example 6: Comparative testing of the toxicity of peptides 13,14, 19 and 4** Toxicity studies of peptides having the sequences 19 and 4 were performed in mice. Each of the peptides were administered intraperitoneally to mice in a 0.1 - 10mg/kg dose (equivalent to 700mg in human). At these high levels, no organ toxicity was observed. Mild neutropenia was detected.

At lower doses (1 and 0.1 mg/kg) no organ toxicity and no neutropenia was detected in mice.

### Example 7: Testing of the activity of peptide 4 on a range of different diseases

The peptide having the sequence 4 was tested to determine its effect on a range of different diseases in humans, horses and cows.

The results of the testing in humans are provided in table 8. The peptides were prepared as compositions containing 1mg of peptide dissolved in 5ml of a solution comprising water, bicarbonate and either benzyl alcohol or methanol.

**TABLE 8: Testing of peptide 4 in human subjects for treatment of a number of different diseases and conditions**

| **Disease** | **Dose** | **Route** | **Effect** | **Duration** |
|---|---|---|---|---|
| Osteoarthritis | 1mg | Joint | Mild | Single 48hrs |
| T1 Diabetes | 1mg | IM | Significant | Single 96hrs |
| Tonsilitis | 1mg | oral | Pronounced | Single 48hrs |
| Lymphodema | 1mg | IM | Pronounced | Single 96hrs |
| Emphysema | 1mg | SC | Moderate | Single 48hrs |
| Dermatitis | 1mg | SC | Pronounced | Single 96hrs |
| Cancer Pain from: | | | | |
| Lung Cancer | 1mg | SC | Pronounced | Repeated |
| Adrenal Cancer | 1mg | IM | Moderate | Repeated |
| Bone Metasta | 1mg | IM | Pronounced | Repeated |

These results show that 1 mg administration of peptide intra-articularly, intramuscularly, orally, subcutaneously was effective in reducing pain due to inflammation or disease parameters due to inflammation (type 1 diabetes) in a spectrum of disorders in the human subjects tested. Depending on the disorder variability was seen in the extent of reduction of inflammation and duration of effect.

The results of the testing in horses are provided in table 9. The peptides were prepared as compositions containing 1mg of peptide dissolved in 5ml of solution, comprising water, bicarbonate and either benzyl alcohol or methanol.

**TABLE 9: Testing of peptide 4 in horses for treatment of osteoarthritis and equine laminitis**

| **Disease** | **Dose** | **Route** | **Effect** | **Duration** |
|---|---|---|---|---|
| OA | 1mg | Joint | Moderate | 48hrs |
| Laminitis | 1mg | IV | Pronounced | 72hrs |

These results show that administration of 1 mg of peptide 4 intra-articularly into osteoarthritis joints of horses and intravenously in horses suffering from laminitis was effective in reducing pain due to inflammation or disease parameters due to inflammation. The positive effect on horse laminitis was pronounced compared to the moderately positive effect observed with intra-articular injection of osteoarthritic joints.

The results of the testing in cows are provided in table 10. The peptides were prepared as compositions containing 1mg of peptide dissolved in 5ml of a solution comprising water, bicarbonate and either benzyl alcohol or methanol.

**TABLE 10: Testing of peptide 4 in cows for treatment of mastitis**

| **Disease** | **Dose** | **Route** | **Effect** | **Duration** |
|---|---|---|---|---|
| Mastitis | 1mg | IV | Pronounced | 72hours |

These results show that a very pronounced effect was observed in inflammation due to mastitis in cows after intravenous administration of peptide 4.

### Example 8: Equine Case Studies

In these case studies, 13 different horses each suffering from different inflammatory conditions were treated with peptide 4, which was administered by injection in a composition containing the peptide dissolved in 5ml of a solution comprising water, bicarbonate and either benzyl alcohol or methanol. The results of each of these case studies is summarised below.

| **Case** | **Age (years)** | **Condition** | **Peptide administered** | **Observations** |
|---|---|---|---|---|
| 1 | 8 | Severe inflammation of the near hind outer branch of the suspensory ligament. | 1 mg of peptide 4 by bolus intravenous injection. | No inflammation was observed for 48 hours post-administration. |
| 2 | 4 | Severe pain and heat in the near fore hoof. | 1 mg of peptide 4 by bolus intravenous injection. | Minimal pain and no heat were observed in the hoof over a period of 48 hours post-administration. |
| 3 | 7 | History of years of mild laminitis, as seen by pulse, heat and varying pain levels in both front hooves. | 1 mg of peptide 4 by bolus intravenous injection every 3^{rd} day for three weeks. | Complete relief of all symptoms and pain was observed during period of treatment. |
| 4 | 3 | Near fore fetlock with constant heat and pain on flexion, and severe fluid build up in the lower deep flexor tendon. | 1 mg of peptide 4 by bolus intravenous injection. | Fetlock completely settled down and no fluid deposit behind pastern was observed after 8 days. 10 days post-administration symptoms returned, but after further treatment the same result was achieved. |
| 5 | 2 | Severe gluteal tear on the lateral side. | 0.33mg of peptide 4 was injected intramuscularly at 3 places around the tear. | No pain or palpation was observed for 14 days. |
| 6 | 7 | History of four years of general knee joint pain. | Sodium hyaluranate (Synvisc®) intra-articularly into both knees followed by 0.5mg of peptide 4 at the same location. | Complete pain relief along with increased flexion of the joint was observed for 6 days. |
| 7 | 6 | History of three years of general knee pain. | Sodium hyaluranate (Synvisc®) intra-articularly into both knees followed by 0.5mg of peptide 4 at the same location. | Complete pain relief along with increased flexion of the joint was observed. No follow up treatment was required. |
| 8 | 4 | Pain and heat in lateral fore-hoof. | 1 mg of peptide 4 by bolus intravenous injection | Pronounced relief of symptoms was observed over a 72 hour period. |
| 9 | | Severe non-diagnosable muscle/ligament tear between the shoulder and the rib cage. | 1mg of peptide 4 by bolus intravenous injection. | Symptoms reduced within 24 hours and significant improvement was noted for over 3 weeks. |
| 10 | 2 | Chronic joint pain in both knees and fetlocks. | Sodium hyaluranate (Synvisc®) intra-articularly into both knees followed by 0.5mg of peptide 4 at the same location. | Significant pain relief, improved joint function and improvement after strenuous exercise within 24 hours posttreatment. |
| 11 | 2 | Mild work-related shin soreness. | 1mg of peptide 4 by bolus intravenous injection every 3^{rd} day for a week. | Significant improvement was observed after the second injection. |
| 12 | 2 | Mild work-related shin soreness. | 1 mg of peptide 4 by bolus intravenous injection. | Significant pain relief and improvement after strenuous exercise was observed after 24 hours post-administration. |
| 13 | 5 | Severe pain due to inflammation and fluid build-up at the site of a 18 month old tendon injury. | 1 mg of peptide 4 weekly by bolus intravenous injection. | Complete relief of pain, inflammation and fluid build up was observed post-administration. |

### Examples 9 to 28 - Treatment of Cachexia in Cancer patients

Cachexia is a complex metabolic syndrome associated with an underlying illness, and is associated with increased morbidity. This syndrome is characterized by loss of muscle or lean body mass with or without loss of fat. Muscle wasting is an important phenotypic feature of cancer cachexia and is the principle cause of function impairment, fatigue and respiratory complications. Providing additional food and/or nutrients to a patient with cachexia does not reverse the weight loss or other underlying problems. Even with total parenteral nutrition, subjects suffering from cachexia will continue to lose skeletal muscle mass. Accordingly, cachexia is distinct from starvation, age-related loss of muscle mass, primary depression, malabsorption and hyperthyroidism.

In addition to weight loss, patients suffering from cachexia may also experience inflammation, insulin resistance, low levels of testosterone and other anabolic hormones, anaemia, increased muscle protein breakdown and/or growth failure in children.

Patients are typically diagnosed as having cachexia where weight loss of at least 5% in 12 months or less (or BMI <20kg/m²), is accompanied by at least three of the following conditions:
- Decreased muscle strength
- Fatigue
- Low fat-free mass index; and
- Abnormal blood biochemistry: increased inflammatory markers (CRP, IL-6), anaemia (Hb <12g/dL) and low serum albumin (<3.2 g/dL)

Existing therapeutic strategies for treating cachexia include the administration of steroid drugs. However, these drugs have not been able to effectively treat and/or alleviate the symptoms of cachexia.

The peptides and compositions of the present invention are suitable for the treatment of cachexia.

Generally, the term "treating" means affecting a subject, tissue or cell to obtain a desired pharmacological and/or physiological effect and includes: (a) the peptide binding to and/or acting as an antagonist of IL-1R to alleviate the symptoms of cachexia; (b) relieving or ameliorating the effects of cachexia; (c) reducing the incidence of cachexia, or (d) preventing cachexia from occurring in a subject, tissue or cell predisposed to cachexia, or at risk thereof (but not yet been diagnosed with), by providing a protective pharmacological and/or physiological effect so that the cachexia does not develop or occur. Without wishing to be bound by theory it has been hypothesised that treatment of cachexia using the peptides of the present invention is achieved by the peptide binding to and/or acting as an antagonist of IL-1R, and treating the inflammatory component of cachexia.

Cachexia may be associated with a number of underlying illnesses. The underlying illness may be a terminal illness, or a condition which requires palliative care. Example of the types of underlying illnesses include cancer, AIDS, chronic obstructive lung disease, congestive heart failure, tuberculosis, familial amyloid polyneuropathy, mercury poisoning (acrodynia) and hormonal deficiency. In addition, cachexia may be seen in subjects with drug addictions, such as subjects who abuse methamphetamine.

Cancer cachexia is a debilitating and life-threatening syndrome characterised by weight loss, including the loss of both adipose tissue and skeletal muscle. Not only does cancer cachexia impair the quality of life of cancer patients, but it also reduces the patients response to anticancer therapies such as chemotherapy and radiation therapy. This can increase the morbidity and mortality of cancer patients. Cachexia is often ultimately responsible for the death of cancer patients.

The following examples show treatment of cachexia in cancer patients, however, it will be appreciated that the peptides of the present invention could equally be used for treatment of cachexia in patients suffering from another undelying illness or condition.

### Example 9: BREAST CANCER

A female patient who had previously been diagnosed with breast cancer 6 years ago, complained of a sore right hip which had been painful for a week. There had been no history of trauma to the hip, and x-ray showed that the hip was normal. The patient had previously received a short course of radiotherapy and chemotherapy, had declined surgery, and had tried alternative treatments in Mexico.

Bony metastases were considered to be the likely cause of hip pain. The patient had been prescribed a non-steroidal anti-inflammatory (Ibuprofen, 400 mg, twice per day) and paracetamol (one dose administered at night).

A bone scan confirmed an isolated metastasis in the right hip. After some reluctance, the patient commenced taking morphine (an oral morphine mixture, 5-10 mg, 4 hourly) and continued to take Naproxen (Naproxen tablets, 500 mg, daily). On experiencing 'breakthrough pain', the patient was given morphine (morphine mixture, 5 mg, orally, as needed). This breakthrough dose was prescribed for night time and was an important strategy in pain management. A dose of sustained release morphine was also given (20 mg, twice daily).

In the past, the patient had experienced nausea from both pethidine (given during labour) and panadene forte, therefore a prophylactic anti-emetic was prescribed when treatment with morphine was initiated. A prophylactic laxative was also prescribed to prevent the side effect of constipation.

The patient was then prescribed a higher dosage of morphine (10mg morphine mixture 4 hourly at 0630, 1030, 1430 and 1830, with a double dose administered at 2230 to keep the patient free of pain overnight), with four additional doses of 5mg morphine mixture administered over a 24 hour period.

The patient then underwent a further two week course of radiotherapy. On the last day the two-week radiotherapy course, the patient became progressively drowsy, and mildly nauseated by morphine (80mg Kapanol, daily without a prescribed anti-emetic). A physical examination revealed the following:
- Right hip pain virtually gone;
- Small pupils; and
- Decreased respiratory rate.

The patient had symptoms of morphine overdose, as her daily morphine requirement had reduced due to the analgesic effect of palliative radiotherapy (which usually takes 2-3 weeks to occur). The patients daily dose of morphine was therefore reduced, and her daily dose of morphine stabilised (Kapanol 20mg, administered as a bolus dose).

The patient subsequently developed intractable nausea, confusion and drowsiness. These symptoms were assessed as being opioid related, after excluding other causes (such as brain metastases, hypercalcaemia and renal failure). Three different management options were available at this point:
1. Reduce the dose of morphine ;
2. Change the route of morphine administration (eg. from oral to continuous subcutaneous infusion); or
3. Change morphine to a different opioid (opioid substitution)
The patient did not want to have a syringe driver at this stage (option 2), and therefore an opioid substitution (option 3) was selected to improve any adverse side effect(s) while maintaining an equivalent dose of analgesia.

Oxycodone was felt to be an appropriate alternative to morphine. Oxycodone is available in a sustained release formulation called oxycontin (in the form of 10 mg, 20 mg, 40 mg, 80 mg tablets, administered as a bolus dose). The conversion ratio of morphine to oxycodone is 1:1, and therefore the morphine (Kapanol 40 mg administered as a bolus dose) could be changed to oxycodone (Oxycontin 40 mg administered as a bolus dose).

In addition to pain management, the treatment of this patient also included a disfiguring, malodorous, weeping, infected fungating tumour of her right breast. Dressings were changed daily, which relieved the pain and odour.

Several months later an unscheduled home visit was conducted because the patient had a four day history of increasing confusion, anorexia, nausea, vomiting and generalised aches and pains. Physical examination of the patient revealed the following:
- Confusion and agitation
- A mini-mental state examination was conducted to assess the cognitive mental status of the patient, including orientation, attention, immediate and short-term recall, language and the ability to follow simple verbal and written commands. This examination gave a result of 18/30 (normal range 24-30/30).
- Clinically 5% dehydrated
- No abdominal tenderness
- Mild hyporeflexia and hypotonia but no lateralising neurological deficit.
- Symptoms indicating that the patient had cachexia.

The patient was then admitted to a palliative care unit for symptom control and respite. After blood pathology, the diagnosis was that the patient was suffering hypercalcaemia, mild renal impairment and liver metastases.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day for two weeks, in a composition containing the peptide dissolved in 5ml of a solution comprising water, bicarbonate and either benzyl alcohol or methanol.

After 24 hours of treatment with the peptide, the cachexia symptoms were alleviated, improvements were seen in the patient's cognitive mental status, and her appetite increased. After this positive response to this treatment the patient was discharged two weeks after entering the palliative care unit, and referred to a home-based palliative care team home. No side effects were noted due to the administration of the peptide.

Several weeks later the patient developed a significant left hemiparesis and left hemianopia secondary to cerebral metastases. The patient was admitted to hospital where her symptoms were treated with radiotherapy and dexamethasone. She was subsequently discharged to a local nursing home and died several months later.

### Example 10: ADRENAL ADENOMAS

A male patient (54 years old) presented with bi-lateral adrenal adenomas, producing very high levels of adrenal hormones. No treatment was available for the treatment of the cancer itself, and the oncologist referred the patient to an endocrinologist to help with the symptoms. The patient was also showing symptoms of cachexia, including weight loss, decreased muscle strength, fatigue and abnormal biochemistry.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day for one month, in a composition containing the peptide dissolved in 5ml of a solution comprising water, bicarbonate and either benzyl alcohol or methanol

After commencement of treatment with the peptide, the symptoms of cachexia were alleviated. The patient's behaviour, appetite, and fatigue concomitantly improved over the treatment period.

### Example 11: GLIOBLASTOMA

A female patient (63 years old) presented with an unressectable glioblastoma in the brain. The patient was taking high levels of dexamethasone. Although her appetite was normal she suffered from secondary depression, mental disclarity, hyporeflexia, hypotonia and had symptoms of cachexia, including weight loss, fatigue, a low fat-free mass index and decreased muscle strength. The patient was then admitted to a palliative care unit for symptom control and respite. After blood pathology, the diagnosis also showed that the patient has abnormal biochemistry and was suffering hypercalcaemia, mild renal impairment and liver metastases.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day, in a composition containing the peptide dissolved in 5ml of a solution comprising water, bicarbonate and either benzyl alcohol or methanol.

After commencement of treatment with the peptide, the symptoms of cachexia were alleviated and the patient's mental clarity improved, and after 24 hours she was able to walk short distances unassisted. The patient has since commenced radiotherapy.

### Example 12: BRAIN ADENOMA

A male patient (57 years old) presented with advanced adenoma in his brain. His vision was affected in that it was blurry and could not recognize the colour red anymore. The patient suffered from secondary depression, and symptoms of cachexia including decreased muscle strength and weight loss.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day for two months, in a composition containing the peptide dissolved in 5ml of a solution comprising water, bicarbonate and either benzyl alcohol or methanol.

Two days after commencement of treatment with the peptide, the patient began feeling less depressed, his eyesight had improved, and his symptoms of cachexia were alleviated.

### Example 13: BRAIN GLIOMA

A female patient (16 year old) was diagnosed with an advanced glioma in the brain. The oncologist did not prescribe any chemotherapy or radiation, as it was believed that the glioma was untreatable. The patient was also showing symptoms of cachexia, namely, abnormal biochemistry, weight loss, fatigue, a low fat-free mass index, and decreased muscle strength.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day for one week, in a composition containing the peptide dissolved in 5ml of a solution comprising water, bicarbonate and either benzyl alcohol or methanol.

Three days after commencement of treatment with the peptide, the patient's feelings of depression started to diminish, she started getting her appetite back and mental clarity had also improved. The patient's symptoms of cachexia were alleviated. One week later she was transferred out of a terminally ill centre into a physical therapy centre.

### Example 14: OVARIAN CANCER

A female patient (33 years old) presented with ovarian cancer with metastasis to the brain (a low grade left frontal glioma) and in the liver between the anterior and posterior segments. The patient was also showing symptoms of cachexia, namely, abnormal biochemistry, weight loss, fatigue, a low fat-free mass index, and decreased muscle strength.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4)

The peptide was administered by subcutaneous injection every second day for one month, in a composition containing the peptide dissolved in 5ml of a solution comprising water, bicarbonate and either benzyl alcohol or methanol.

After 24 hours from commencement of treatment with the peptide, the patient's feelings of depression had markedly reduced and the patient continued to improve throughout the month of treatment. The patient's symptoms of cachexia were also alleviated.

### Example 15: BONE CANCER

A female patient (66 years old) had a 9 year history of bone cancer, and was advised by her oncologist that her condition was not treatable. Nine vertebrae had already collapsed, and the patient's bones had severe osteopenia where a small pressure as a bump against the ribs could crack a rib. The cancer had spread through the bones in the spine in such a way that her life expectancy was estimated to be 3 to 6 months with increasing pain. The patient suffered from severe muscle cramps and pain across the shoulders and chest. The patient was administered large doses of morphine, oxycontin and paracetamol during pain attacks. This gave side effects of nausea and constipation, which were difficult to endure. The patient had lost 20 kg in weight as her appetite was suppressed, and was suffering from these and other symptoms of cachexia.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day for one month, in a composition containing the peptide dissolved in 5ml of a solution comprising water, bicarbonate and either benzyl alcohol or methanol.

Two days after commencement of treatment with the peptide, the patient's appetite and depression started to improve. The patient also noticed that she was taking less breakthrough pain medication. The patient's symptoms of cachexia were alleviated.

### Example 16: BREAST CANCER

A female patient (Age: 51; Weight 65kg) presented with metastasized stage 4 breast cancer to the bone and cancer in vertebrae and right humerus. The year before the patient had been diagnosed with stage 2b cancer where lumpectomy, chemotherapy and radiation therapy was performed. The patient suffered from neck shoulder and back pain and was on a fentanyl patch for the neck pain. The patient also had type II diabetes, and was showing symptoms of cachexia.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day for one month, in a composition containing the peptide dissolved in 5ml of a solution comprising water, bicarbonate and either benzyl alcohol or methanol.

The patient reported that her appetite (she had put on 5kg), and her mental demeanour had markedly improved. The patient's symptoms of cachexia were also alleviated.

### Example 17: BREAST CANCER

A female patient (53 years old) had undergone a total mastectomy for breast cancer in the previous year and post-resection chemotherapy, as well as hormone therapy (5 year treatment). The patient presented with marked weight loss, depression, muscular weakness and fatigue, and was suffering from cachexia.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day for two weeks, in a composition containing the peptide dissolved in 5ml of a solution comprising water, bicarbonate and either benzyl alcohol or methanol.

One week after commencement of treatment with the peptide, the patient had much more energy and her appetite had improved. Two weeks later the patient had continued to improve with her entire mental and physiological situation being vastly improved. The patient's symptoms of cachexia were alleviated.

### Example 18: BLADDER CANCER

A male patient (71 years old) presented with bladder cancer. The patient frequently had blood in his urine and was examined by a urologist who found a tumour at the base of the bladder near the urethra. The patient subsequently had the tumour removed by cystoscopy and pathology showed it was a papillary transitional cell carcinoma grade 3/3 invasive into subepithelial connective tissue.

Over 6 months the patient tried some complementary/alternative cancer treatments, and during this time he was still experiencing blood in his urine, which was starting to become quite frequent. A rectal MRI showed a large tumour near the urethra.

The patient declined surgery to have his bladder removed, but underwent chemotherapy for four months. After a CT scan the tumour was shown to have reduced by 2/3 in size. At this point the patient had developed severe cachexia and chemotherapy treatment was stopped. Conventional treatment for cachexia was started which consisted of dexamethasone daily and parenteral nutrition which over one month had little effect.

Following this the patient was treated with a peptide according to the invention, which involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day for one month in a composition containing the peptide dissolved in 5ml of a solution comprising water, bicarbonate and either benzyl alcohol or methanol.

After 24 from the commencement of treatment with the peptide, symptoms of cachexia were alleviated. The patient's appetite had returned and his entire demeanour had changed in a positive way. The patient continued to improve over the month of therapy, and has since resumed chemotherapy.

### Example 19: COLON CANCER

A male patient (43 years old) presented with colon cancer and a 30 year history of inflammatory bowel syndrome. The patient was experiencing a severe sharp pain each time he had a bowel movement, low appetite, fatigue, depression and sleeplessness. The patient was also showing symptoms of cachexia, namely, abnormal biochemistry, weight loss, a low fat-free mass index, fatigue and decreased muscle strength.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day for one month, in a composition containing the peptide dissolved in 5ml of a solution comprising water, bicarbonate and either benzyl alcohol or methanol.

Over the first week of initiating treatment, the patient's insomnia and depression had significantly decreased as well as a marked improvement in appetite and muscular strength. The symptoms of cachexia were largely alleviated.

### Example 20: COLON CANCER

A male patient (42 years old) presented with colon cancer. This patient had undergone surgery, chemotherapy and radiation six months prior. The patient was very fatigued, bleeding intermittently from the bowel, was in excruciating pain due to the multiple bone metastases, and was suffering from cachexia.

Treatment of the patient with a peptide according to the invention was initiated, and involved administration of 1 mg of a peptide having the following sequence:

AC-DLeu-DPro-DLeu-DAla-DTyr-DPro-DGIn-DTrp-DTyr-DGly-DPro-DThr-DTrp-DGlu-DPhe (SEQ ID NO: 4).

The peptide was administered by subcutaneous injection every second day for one month, in a composition containing the peptide dissolved in 5ml of a solution comprising water, bicarbonate and either benzyl alcohol or methanol.

After 48 hours from the commencement of treatment, the patient had a meal without experiencing post-nausea and vomiting. One month after commencement of treatment with the peptide, the patient was in great shape, and the symptoms of cachexia had been treated.

### Example 21: GLIOBLASTOMA

A male patient (48 years old) was taken to the emergency room of the local hospital with a splitting headache and projectile vomiting. After a scan it was discovered to be a brain tumour. In the same month the patient had surgery and subsequent tests later confirmed that he had a Glioblastoma Multiforme IV. The surgeon removed all the visible tumour tissue. However, the patient was told that he had about 26 weeks to live, and with radiation therapy he may have an extra 12 months of life. After 5 days of radiation therapy, the patient began to suffer from cachexia. Due to his subsequent weakness radiation therapy was stopped.

Conventional treatment for cachexia was started which consisted of dexamethasone daily and parenteral nutrition which over one month had little effect.

Following this the patient was treated with a peptide according to the invention, which involved administration of 1 mg of a peptide having the following sequence:

AC-DLeu-DPro-DLeu-DAla-DTyr-DPro-DGIn-DTrp-DTyr-DGly-DPro-DThr-DTrp-DGlu-DPhe (SEQ ID NO: 4).

The peptide was administered by subcutaneous injection every second day for one month, in a composition containing the peptide dissolved in 5ml of a solution comprising water, bicarbonate and either benzyl alcohol or methanol.

Two days after commencement of treatment with the peptide, the patient's cachexia started to diminish. After one month of treatment with the peptide, the patient was fit and confident to start radiation therapy again.

### Example 22: GLIOBLASTOMA

A male patient (54 years of age) was diagnosed 3 weeks previously with Glioblastoma Multiforme, and was hospitalized 3 days prior to presenting when his condition had suddenly deteriorated. The patient remained in hospital and was unable to move half of his body. The patient had edema in his brain, could hardly eat, and was unable to talk. Radiation and dexamethasone was prescribed to try and reduce the edema and it was postulated that the patient would not recover from such a grave condition. The patient was also showing symptoms of cachexia, namely, abnormal biochemistry, weight loss, fatigue and decreased muscle strength.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day, in a composition containing the peptide dissolved in 5ml of a solution comprising water, bicarbonate and either benzyl alcohol or methanol.

After 24 hours from commencement of treatment with the peptide, the symptoms of cachexia had been alleviated. After two weeks from commencement of treatment with the peptide, this improvement was maintained, and the patient has been able to start radiation therapy.

### Example 23: HODGKINS LYMPHOMA

A male patient (7 years old) presented with Hodgkin's Lymphoma. The patient underwent surgery two years prior, followed by radiation therapy and additional experimental therapies under clinical trial format without success. The patient was discharged, and the condition was considered to be incurable, with an estimated life expectancy of less than six months. The patient's voice was hoarse (vocal cord damage from radiation) and his breathing laboured, and he also showed symptoms of cachexia.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day for one month, in a composition containing the peptide dissolved in 5ml of a solution comprising water, bicarbonate and either benzyl alcohol or methanol.

After three weeks from commencement of treatment with the peptide, the symptoms of cachexia were alleviated, and the patient was able to return to school after 1 month.

### Example 24: COLON CANCER

A female patient (62 year old) presented with stage IV (terminal) colorectal cancer, with metastases in the liver and lung. The patient suffered from extreme cachexia before her oncologist indicated that there was no more that could be done. The patient was receiving a cocktail of pain medication.

Treatment of the patient with a peptide according to the invention was initiated, and involved administration of 1 mg of a peptide having the following sequence:

AC-DLeu-DPro-DLeu-DAla-DTyr-DPro-DGIn-DTrp-DTyr-DGly-DPro-DThr-DTrp-DGlu-DPhe (SEQ ID NO: 4).

The peptide was administered by subcutaneous injection every second day, in a composition containing the peptide dissolved in 5ml of a solution comprising water, bicarbonate and either benzyl alcohol or methanol.

After two weeks from commencement of treatment with the peptide, there was a marked improvement in her restlessness, nausea and vomiting and appetite suppression, and the symptoms of cachexia were alleviated. The patient has also has gained closed to 5 kg in weight.

### Example 25: NON-HODGKIN'S LYMPHOMA

A male patient (42 years old) who had non Hodgkin's Lymphoma since he was 17 yrs old presented with his fifth reoccurrence of his disease. The patient had been receiving various chemotherapy treatments, radiation and cobalt and surgery in the past. The patient was told by his oncologist that as he grew older the cancer would show up in places that would be more difficult to treat.

When he presented, the patient's cancer had severely affected his left leg, growing in amongst the muscles and tendons and nerves so surgery was not an option, while the patient refused to have any more chemotherapy. The patient's ability to walk was extremely hampered, was in constant pain, and was basically house bound. The patient had also started to develop cachexia.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day, in a composition containing the peptide dissolved in 5ml of a solution comprising water, bicarbonate and either benzyl alcohol or methanol.

After 4 days from commencement of treatment with the peptide, the patient also regained his appetite, and was found to feel very little depression and no lethargy. The patient's symptoms of cachexia were alleviated.

### Example 26: PANCREATIC CANCER

A female patient (52 years old) was diagnosed with pancreatic cancer one year prior. The patient had the tumour surgically removed and her prognosis was good.

Subsequently, the patient was diagnosed with multiple metastases in the lungs and liver. The patient had lost close to 30 kg within three months and was suffering from severe cachexia. The patient was not fit for chemotherapy treatment, and was taking paracetamol every 4 hours for her pain.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day, in a composition containing the peptide dissolved in 5ml of a solution comprising water, bicarbonate and either benzyl alcohol or methanol.

After 24 hours from commencement of treatment with the peptide, the patient's appetite and clarity of mind improved. After 7 days of treatment the patient was consistently eating with no nausea or vomiting, feeling less depressed and less lethargic. The patient's symptoms of cachexia were alleviated.

On ceasing treatment with the peptide, the symptoms of cachexia began to set back in, and this condition progressively worsened. When treatment with the peptide resumed, the patient's cachexic state again started to diminish, and this occurred within 24 hours.

### Example 27: LIPOSARCOMA

A male patient (64 years old) was suffering from a large abdominal tumour (liposarcoma abdominal) and moderate cachexia. The patient's tumour was 50cm x 35 cm and when surgically removed weighed 14 kg. Although there was no sign of further tumour growth or metastases after four months from the operation, the patient still suffered from cachexia, despite numerous conventional and alternative treatments for this.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day, in a composition containing the peptide dissolved in 5ml of a solution comprising water, bicarbonate and either benzyl alcohol or methanol.

After 3 days from commencement of treatment with the peptide, the patient noticed a remarkable improvement in his appetite, depression, fatigue and mental clarity, and the symptoms of cachexia were alleviated.

### Example 28: LIVER CANCER

A female patient (72 years of age) with terminal liver cancer presented with severe cachexia. She was very weak could barely move from her wheelchair.

Treatment of the patient with a peptide according to the invention was initiated, and involved administration of 1 mg of a peptide having the following sequence:

AC-DLeu-DPro-DLeu-DAla-DTyr-DPro-DGln-DTrp-DTyr-DGly-DPro-DThr-DTrp-DGlu-DPhe (SEQ ID NO: 4).

The peptide was administered by subcutaneous injection every second day, in a composition containing the peptide dissolved in 5ml of a solution comprising water, bicarbonate and either benzyl alcohol or methanol.

After one week from the commencement of treatment with the peptide, the patient's had started to take slow walks, her appetite had markedly improved and her pain had decreased with less use of oxycontin. Upon observation her demeanour had positively changed, and the patient's depression seemed to have decreased. The patient also started eating again. The symptoms of cachexia were largely alleviated.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

## Claims

1. A peptide comprising the sequence of general formula (III): 
Z1-X20-DLeu-DPro-DLeu-DAla-DTyr-DPro-DGln-DTrp-DTyr-DGly-DPro-DThr-DTrp-DGlu-DPhe-
X21-Z2
(III)
 or salts, solvates or tautomers thereof, wherein X₂₀ and X₂₁, are each independently absent or one or more amino acids; and Z₁ and Z₂ are each independently absent or a protecting group.

2. The
peptide according to claim 1 or salts, solvates, or tautomers thereof, wherein X₂₀ and X₂₁ are absent and Z₁ and Z₂ are each independently absent or a protecting group.

3. The peptide according to claim 1 or 2, wherein Z₁ is selected from the group consisting of acetyl, Fmoc, Troc, Boc, Alloc, Teoc and Cbz.

4. The peptide according to any one of claims 1 to 3, wherein Z₂ is selected from the group consisting of an alkyl ester and a benzyl ester.

5. The peptide according to claim 1, wherein the peptide is of the sequence of general formula (IV):
Z₁-DLeu-DPro-DLeu-DAla-DTyr-DPro-DGln-DTrp-DTyr-DGly-DPro-DThr-DTrp-DGlu-DPhe (IV)
or salts, solvates, or tautomers thereof.

6. The peptide according to claim 5, wherein Z₁ is an acetyl protecting group

7. An IL-1 receptor antagonist comprising the peptide according to any one of the claims 1 to 6, or a salt, solvate or tautomer thereof.

8. A pharmaceutical composition comprising the peptide according to any of the claims 1 to 6, or a salt, solvate or tautomer thereof and a pharmaceutically acceptable carrier.

9. The peptide according to any one of claims 1 to 6, or a salt, solvate or tautomer thereof, for use in the treatment of an IL-1 related disease or condition.

10. The peptide for use according to claim 9, wherein the IL-1 related disease or condition is selected from the group consisting of inflammation, autoimmune diseases and immune diseases or a disease or condition comprising an inflammatory, autoimmune or immune component.

11. The peptide for use according to claim 9 or claim 10, wherein the IL-1 related disease or condition is selected from the group consisting of acne vulgaris, acute and chronic lung disease, acute and chronic interstitial lung disease, inflammatory lung disease, acute febrile illness due to bacteria or viruses, acute myocardial infarction, alcoholic cirrhosis, anorexia, asthma, atherosclerosis, arthritis and rheumatic diseases, rheumatoid arthritis, arthritis chronica progrediente, arthritis deformans, juvenile arthritis, spondylarthritis, asbestosis, autoimmune disease, autoimmune hematological disorders, autoimmune cardiomyopathy, autoimmune hepatitis, autoimmune pancreatitis, autoimmune polyendocrine syndrome, autoimmune progesterone dermatitis, autoimmune thrombocytopenic purpura, autoimmune urticaria, autoimmune uveitis, autoimmune inflammatory bowel disease, ulcerative colitis, distal colitis, Crohn's disease, bacterial infection, burns, cachexia, cancer, cardiopulmonary bypass, celiac disease, cerebral infarction, cerebral palsy, chronic active hepatitis, chronic prostatitis, chronic renal failure, coal miner pneumonoconiosis, contact dermatitis, cuprophane hemodialysis, dermatitis, dermatomyositis, diabetes, juvenile diabetes, insulin diabetes, diabetes mellitus, type I diabetes, endocrine ophthalmopathy, eosinophilic fasciitis, equine laminitis, glomerulonephritis, gout and pseudogout, graft versus host disease, Graves disease, Gullain-Barré syndrome, Hashimoto's encephalitis, Hashimoto's thyroiditis, hemophagocytic lymphohistiocytosis, HIV infection and AIDS, hypersensitivities, idiopathic spure, inflammatory bowel disease, interstitial lung fibrosis, ischemia injury, Kawasaki's disease, leukemia, myelogenous leukemia, lupus erythematosus, luteal phase defect, Miller-Fisher syndrome, mixed connective tissue disease, keratoconjunctivitis sicca, vernal keratoconjunctivitis, mastitis, multiple sclerosis, myasthenia gravis, obstructive jaundice, osteomalacia, osteoarthritis, osteoporosis, pain, Paget's disease, pediatric sepsis syndrome, pelvic inflammatory disease, polychondritis, polymyositis, pre-eclampsia, primary biliary cirrhosis uveitis, psoriasis, psoriatic arthritis, pulmonary hypertension, relapsing polychondritis, reperfusion injury, respiratory distress syndrome, reticulohistiocytosis, sarcoidosis, scleroderma, sepsis, septic shock, Sjögren's syndrome, silicosis, Steven-Johnson syndrome, systemic lupus erythematosus, temporal arteritis, thermal injury, toxic shock syndrome, transplant rejection, tuberculosis, undifferentiated connective tissue disease, vasculitis and intestinal cystitis and Wegener granulamatosis.

12. The peptide according to any one of claims 1 to 6, or a salt, solvate or tautomer thereof, for use in therapy.

13. The peptide for use according to claim 12, wherein said use is inhibiting the effect of IL-1.

## Patentansprüche

1. Peptid, umfassend die Sequenz der allgemeinen Formel (III): 
Z1-X20-DLeu-DPro-DLeu-DAla-DTyr-DPro-DGln-DTrp-DTyr-DGly-DPro-DThr-DTrp-
DGlu-DPhe-X21-Z2
(III)
 oder Salze, Solvate oder Tautomere davon, worin X₂₀ und X₂₁ jeweils unabhängig nicht vorliegen oder eine oder mehrere Aminosäuren sind; und Z₁ und Z₂ jeweils unabhängig nicht vorliegen oder eine Schutzgruppe sind.

2. Peptid nach Anspruch 1 oder Salze, Solvate oder Tautomere davon, worin X₂₀ und X₂₁ nicht vorliegen und Z₁ und Z₂ jeweils unabhängig nicht vorliegen oder eine Schutzgruppe sind.

3. Peptid nach Anspruch 1 oder 2, worin Z₁ ausgewählt ist aus der Gruppe, bestehend aus Acetyl, Fmoc, Troc, Boc, Alloc, Teoc und Cbz.

4. Peptid nach einem der Ansprüche 1 bis 3, worin Z₂ ausgewählt ist aus der Gruppe, bestehend aus einem Alkylester und einem Benzylester.

5. Peptid nach Anspruch 1, worin das Peptid die Sequenz der allgemeinen Formel (IV) aufweist: Z1-DLeu-DPro-DLeu-DAla-DTyr-DPro-DGln-DTrp-DTyr-DGly-DPro-DThr-DTrp-DGlu- 
ᴅPhe
(IV)
 oder Salze, Solvate oder Tautomere davon.

6. Peptid nach Anspruch 5, worin Z₁ eine Acetyl-Schutzgruppe ist.

7. IL-1-Rezeptor-Antagonist, umfassend das Peptid nach einem der Ansprüche 1 bis 6 oder ein Salz, Solvat oder Tautomer davon.

8. Pharmazeutische Zusammensetzung, umfassend das Peptid nach einem der Ansprüche 1 bis 6 oder ein Salz, Solvat oder Tautomer davon und einen pharmazeutisch verträglichen Träger.

9. Peptid nach einem der Ansprüche 1 bis 6 oder ein Salz, Solvat oder Tautomer davon zur Verwendung bei der Behandlung einer Krankheit oder eines Zustands, die mit IL-1 in Beziehung stehen.

10. Peptid zur Verwendung nach Anspruch 9, wobei die mit IL-1 in Beziehung stehende Krankheit oder der mit IL-1 in Beziehung stehende Zustand ausgewählt ist aus der Gruppe, bestehend aus Entzündung, Autoimmunkrankheiten und Immunkrankheiten oder einer Krankheit oder einem Zustand, umfassend eine Entzündung-, Autoimmun- oder Immunkomponente.

11. Peptid zur Verwendung nach Anspruch 9 oder Anspruch 10, wobei die mit IL-1 in Beziehung stehende Krankheit oder der mit IL-1 in Beziehung stehende Zustand ausgewählt ist aus der Gruppe, bestehend aus Akne vulgaris, akute und chronische Lungenerkrankung, akute und chronische interstitielle Lungenerkrankung, entzündliche Lungenerkrankung, akute Fieber-Krankheit aufgrund von Bakterien oder Viren, akuter Myokardinfarkt, alkoholische Zirrhose, Anorexie, Asthma, Atherosklerose, Arthritis und rheumatische Erkrankungen, rheumatoide Arthritis, progrediente chronische Arthritis, Arthritis deformans, juvenile Arthritis, Spondylarthritis, Asbestose, Autoimmunkrankheit, autoimmune hämatologische Störungen, autoimmune Kardiomyopathie, Autoimmunhepatitis, Autoimmunpankreatitis, polyendokrines Autoimmunsyndrom, Autoimmun-Progesteron-Dermatitis, autoimmune thrombozytopenische Purpura, Autoimmun-Urtikaria, autoimmune Uveitis, autoimmune entzündliche Darmerkrankung, Colitis ulcerosa, distale Colitis, Morbus Crohn, bakterielle Infektion, Verbrennungen, Kachexie, Krebs, kardiopulmonaler Bypass, Zöliakie, Hirninfarkt, Zerebralparese, chronisch aktive Hepatitis, chronische Prostatitis, chronisches Nierenversagen, Kohlenbergmann-Pneumonie, Kontaktdermatitis, Cuprophan-Hämodialyse, Dermatitis, Dermatomyositis, Diabetes, juvenile Diabetes, Insulin-Diabetes, Diabetes mellitus, Typ-I-Diabetes, endokrine Ophthalmopathie, eosinophile Fasziitis, Pferde-Laminitis (Hufrehe), Glomerulonephritis, Gicht und Pseudogicht, Graft-versus-Host-Krankheit, Graves-Krankheit, Gullain-Barre-Syndrom, Hashimoto-Enzephalitis, Hashimoto-Thyroiditis, hämophagozytische Lymphohistiozytose, HIV-Infektion und AIDS, Überempfindlichkeitsreaktionen, idiopathische Sprue, entzündliche Darmerkrankung, interstitielle Lungenfibrose, Ischämieverletzung, Kawasaki-Krankheit, Leukämie, myeloische Leukämie, Lupus erythematodes, Lutealphasendefekt, Miller-Fisher-Syndrom, gemischte Bindegewebserkrankung, Keratokonjunktivitis sicca, vernale Keratokonjunktivitis, Mastitis, Multiple Sklerose, Myasthenia gravis, obstruktiver Ikterus, Osteomalazie, Osteoarthritis, Osteoporose, Schmerz, Morbus Paget, pädiatrisches Sepsis-Syndrom, Entzündungen des Beckens, Polychondritis, Polymyositis, Präeklampsie, primäre Gallenwege-Zirrhose Uveitis, Psoriasis, Psoriasis-Arthritis, pulmonale Hypertonie, Rückfall-Polychondritis, Reperfusionsverletzung, Atemnotsyndrom, Retikulohistiozytose, Sarkoidose, Sklerodermie, Sepsis, septischer Schock, Sjögren-Syndrom, Silikose, Steven-Johnson-Syndrom, systemischer Lupus erythematodes, Arteriitis temporalis, thermische Verletzung, toxischer Schock-Syndrom, Transplantatabstoßung, Tuberkulose, undifferenzierte Bindegewebserkrankung, Vaskulitis und Darmzystitis und Wegener-Granulamatose.

12. Peptid nach einem der Ansprüche 1 bis 6 oder ein Salz, Solvat oder Tautomer davon zur Verwendung in der Therapie.

13. Peptid zur Verwendung nach Anspruch 12, wobei die Verwendung Inhibieren der Wirkung von IL-1 ist.

## Revendications

1. Peptide comprenant la séquence de formule générale (III) : 
Z1-X20-DLeu-DPro-DLeu-DAla-DTyr-DPro-DGln-DTrp-DTyr-DGly-DPro-DThr-DTrp-DGlu-DPhe-
X21-Z2
(III)
 ou ses sels, solvates ou tautomères, dans laquelle X₂₀ et X₂₁ sont chacun indépendamment absents ou représentent un ou plusieurs acide(s) aminé(s) ; et Z₁ et Z₂ sont chacun indépendamment absents ou représentent un groupe protecteur.

2. Peptide selon la revendication 1 ou ses sels, solvates ou tautomères, dans lequel X₂₀ et X₂₁ sont absents et Z₁ et Z₂ sont chacun indépendamment absents ou représentent un groupe protecteur.

3. Peptide selon la revendication 1 ou 2, dans lequel Z₁ est choisi dans le groupe consistant en l'acétyle, Fmoc, Troc, Boc, Alloc, Teoc et Cbz.

4. Peptide selon l'une quelconque des revendications 1 à 3, dans lequel Z₂ est choisi dans le groupe consistant en un alkylester et un benzylester.

5. Peptide selon la revendication 1, le peptide étant représenté par la séquence de formule générale (IV) :
Z₁-DLeu-DPro-DLeu-DAla-DTyr-DPro-DGln-DTrp-DTyr-DGly-DPro-DThr-DTrp-DGlu-DPhe (IV)
ou ses sels, solvates ou tautomères.

6. Peptide selon la revendication 5, dans lequel Z₁ représente un groupe protecteur acétyle.

7. Antagoniste du récepteur d'IL-1 comprenant le peptide selon l'une quelconque des revendications 1 à 6, ou un de ses sels, solvates ou tautomères.

8. Composition pharmaceutique comprenant le peptide selon l'une quelconque des revendications 1 à 6, ou un de ses sels, solvates ou tautomères et un support pharmaceutiquement acceptable.

9. Peptide selon l'une quelconque des revendications 1 à 6, ou un de ses sels, solvates ou tautomères, destiné à être utilisé dans le traitement d'une maladie ou d'une affection associée à l'IL-1.

10. Peptide destiné à être utilisé selon la revendication 9, dans lequel la maladie ou l'affection associée à l'IL-1 est choisie dans le groupe consistant en une inflammation, des maladies auto-immunes et des maladies immunes ou une maladie ou une affection comprenant un composant inflammatoire, auto-immun ou immun.

11. Peptide destiné à être utilisé selon la revendication 9 ou 10, dans lequel la maladie ou l'affection associée à l'IL-1 est choisie dans le groupe consistant en l'acné vulgaire, la maladie pulmonaire aiguë et chronique, la maladie pulmonaire interstitielle aiguë et chronique, la maladie pulmonaire inflammatoire, la maladie fébrile aiguë due à des bactéries ou à des virus, l'infarctus aigu du myocarde, la cirrhose alcoolique, l'anorexie, l'asthme, l'athérosclérose, l'arthrite et les maladies rhumatismales, la polyarthrite rhumatoïde, l'arthrite chronica progrediente, l'arthrite déformante, l'arthrite juvénile, la spondylarthrite, l'asbestose, la maladie auto-immune, les troubles hématologiques auto-immuns, la cardiomyopathie auto-immune, l'hépatite auto-immune, la pancréatite auto-immune, le syndrome polyendocrinien auto-immun, la dermatite de progestérone auto-immune, le purpura thrombocytopénique auto-immun, l'urticaire auto-immune, l'uvéite auto-immune, la maladie intestinale inflammatoire auto-immune, la rectocolite hémorragique, la colite distale, la maladie de Crohn, l'infection bactérienne, les brûlures, la cachexie, le cancer, le pontage cardiopulmonaire, la maladie coeliaque, l'infarctus cérébral, l'infirmité motrice cérébrale, l'hépatite chronique active, la prostatite chronique, l'insuffisance rénale chronique, la pneumoconiose des mineurs de charbon, la dermatite de contact, l'hémodialyse de cuprophane, la dermatite, la dermatomyosite, le diabète, le diabète juvénile, le diabète insulino-dépendant, le diabète sucré, le diabète de type I, l'ophtalmopathie endocrinienne, la fasciite à éosinophiles, la fourbure équine, la glomérulonéphrite, la goutte et la pseudo-goutte, la maladie de greffe contre hôte, la maladie de Graves, le syndrome de Guillain-Barré, l'encéphalite de Hashimoto, la thyroïdite de Hashimoto, la lymphohistiocytose hémophagocytaire, l'infection par le VIH et le SIDA, les hypersensibilités, la sprue idiopathique, la maladie intestinale inflammatoire, la fibrose pulmonaire interstitielle, la lésion induite par l'ischémie, la maladie de Kawasaki, la leucémie, la leucémie myélogène, le lupus érythémateux, la défaillance de la phase lutéale, le syndrome de Miller-Fisher, la connectivite mixte, la kératoconjonctivite sèche, la kératoconjonctivite printanière, la mastite, la sclérose en plaques, la myasthénie grave, l'ictère rétentionnel, l'ostéomalacie, l'ostéoarthrite, l'ostéoporose, les douleurs, la maladie de Paget, le syndrome de sepsie pédiatrique, la maladie inflammatoire pelvienne, la polychondrite, la polymyosite, la pré-éclampsie, la cirrhose biliaire primitive, l'uvéite, le psoriasis, l'arthrite psoriasique, l'hypertension pulmonaire, la polychondrite récurrente, la lésion de reperfusion, le syndrome de détresse respiratoire, la réticulohistiocytose, la sarcoïdose, la sclérodermie, la sepsie, le choc septique, le syndrome de Sjögren, la silicose, le syndrome de Steven-Johnson, le lupus érythémateux disséminé, l'artérite temporale, la lésion thermique, le syndrome du choc toxique, le rejet de greffe, la tuberculose, la maladie du tissu conjonctif indifférencié, la vascularite et la cystite intestinale et la granulomatose de Wegener.

12. Peptide selon l'une quelconque des revendications 1 à 6, ou un de ses sels, solvates ou tautomères, destiné à être utilisé en thérapie.

13. Peptide destiné à être utilisé selon la revendication 12, dans lequel ladite utilisation inhibe l'effet de l'IL-1.
